(19) Europäisches Patentamt
European Patent Office
Office européen des brevets

(11) EP 2 729 196 B1

(12) # EUROPEAN PATENT SPECIFICATION

(45) Date of publication and mention of the grant of the patent:
**28.12.2016 Bulletin 2016/52**

(21) Application number: **12733487.8**

(22) Date of filing: **07.07.2012**

(51) Int Cl.:
***A61L 29/08*** *(2006.01)* ***A61L 29/16*** *(2006.01)*

(86) International application number:
**PCT/EP2012/063340**

(87) International publication number:
**WO 2013/007666 (17.01.2013 Gazette 2013/03)**

(54) **BALLOON SURFACE COATING**

BALLONOBERFLÄCHENBESCHICHTUNG

REVÊTEMENT SUPERFICIEL POUR BALLONNETS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.07.2011 PCT/EP2011/003564**

(43) Date of publication of application:
**14.05.2014 Bulletin 2014/20**

(73) Proprietor: **Cardionovum Sp.z.o.o.**
**00-834 Warsaw (PL)**

(72) Inventor: **ORLOWSKI, Michael**
**53173 Bonn (DE)**

(74) Representative: **Arth, Hans-Lothar**
**ABK Patent Attorneys**
**Jasminweg 9**
**14052 Berlin (DE)**

(56) References cited:
**EP-A1- 1 916 006**
**EP-A1- 2 243 501**
**DE-U1-202011 002 713**
**US-A1- 2011 152 766**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 729 196 B1

## Description

The present invention relates to catheter balloons coated with a gradient coating comprising at least one active agent and shellac. Moreover the present invention relates to a special method for coating catheter balloons with a pharmacological active agent and the biodegradable composition shellac.

Implantation of vessel grafts such as stents has become a well-established surgical intervention for the treatment of stenosis. In this context, so-called restenosis (recurrent stenosis), i.e. the reocclusion of the vessel is a frequently occurring complication. There's no exact definition of the term restenosis to be found in literature. The most frequently used morphological definition of restenosis defines restenosis as a reduction of the vessel diameter to less than 50% of the normal value subsequent to successful PTA (percutaneous transluminal angioplasty). Said definition describes an empirically determined value and its hemodynamic meaning and association with clinical symptoms lack scientific background. In practice, clinical deterioration in a patient is often considered a sign for the occurrence of restenosis in the previously treated vessel section.

To avoid such problems, a so-called "biological stenting" may be performed using only a coated catheter balloon without any stent, i.e. the vessels are dilated at a constricted site by the dilatation of a coated catheter balloon, wherein, while the catheter balloon is dilated for a short period of time, a sufficient amount of pharmacological agent is transferred to the vessel wall to avoid re-constriction or reocclusion of the vessel due to the dilatation of the vessel and the delivery of active agents.

Nowadays, it is known that active agents can be applied to a balloon catheter with various matrix-substances, including substances such as the terpenoid shellolic acid. The active agents are released during the balloon inflation at the stenosis, in order to penetrate the arterial wall segment, in order to evolve their antiproliferative and anti-inflammatory effects on the smooth muscle cells and to suppress proliferation in the vessel lumen.

Suppression of cellular reactions is mainly accomplished during the first days and weeks by means of preferably antiproliferative, immunosuppressive and/or antiphlogistic agents and their likewise active derivatives /analogues and metabolites.

The international patent application WO 2004/028582 A1 discloses multifold balloons which are coated, especially within the folds, with a composition of a pharmacological agent and a contrast medium. A method for spray coating catheter balloons is described in WO 2004/006976 A1.

Furthermore, we as well as other research groups have found that the hitherto measured paclitaxel concentrations in porcine coronary artery after treatment with prior art paclitaxel coated catheter balloons were not effective in exerting a therapeutic effect on the inhibition of restenosis.

EP2421572 discloses a coating method of a catheter balloon, preferably textured catheter balloons, using a solution of paclitaxel together with shellac in a suitable organic solvent such as acetone, ethyl acetate, ethanol, methanol, DMSO, THF, chloroform, methylene chloride. Said method for loading catheter balloons comprises sequentially application of a solution of paclitaxel and a solution of shellac, which may result in a gradient but not in a controlled gradient such as applied using a gradient mixer. In addition, the method does not result in a vertical and a horizontal gradient. Furthermore, this application does not describe explicitly a gradient or any effect thereof.

The authors of a publication in Circulation 2004, Vol. 110, 810 - 814 demonstrated that catheter balloons coated with pure Paclitaxel did not show any therapeutic effect. A therapeutic effect was only achieved when the Paclitaxel was combined with the contrast agent solution ULTRAVIST®. ULTRAVIST® is a solution of the contrast agent iopromide. The same observation was made by Cremers et al., Clin. Res. Cardiol., 2008, 97 - Suppl.1.

Therefore, it is an objective of the present invention to apply an active agent, especially preferred the active agent paclitaxel, onto a catheter balloon in such a manner that a coating is created which is easily detached from the balloon and can be effectively transferred to the vessel wall so that a therapeutic effect concerning reduction of restenosis can be achieved.

Said objective is solved by the technical teaching of the independent claims. Further advantageous embodiments of the invention result from the dependent claims, the description, the figures and the examples.

Surprisingly it has been found that a catheter balloon comprising a coating with an active agent and shellac, wherein the coating comprises at least one layer, wherein the active agent has both a horizontal concentration gradient and a vertical concentration gradient, wherein for the vertical concentration gradient the concentration of the active agent increases from the surface of the balloon to the top or the surface of the coating, is suited for resolving said objective.

Thus the present invention relates to a catheter balloon comprising a coating with an active agent and shellac, wherein the coating comprises a horizontal and a vertical concentration gradient of the active agent. Thereby the concentration gradient of the active agent is in the layer of shellac as a matrix substance. One of the concentration gradients is referred herein as radial or vertical concentration gradient, because the concentration of the active agent increases from the surface of the balloon to the top or the surface of the coating or in other words the concentration of the active agent decreases from the top of the coating where the concentration is preferably between 90% by weight to 100% by weight to the surface of the catheter balloon where the concentration of the active agent is preferably between 0% by

weight and 10% be weight.

In addition to this vertical concentration gradient a longitudinal or horizontal concentration gradient can be present so that the concentration of the active agent decreases from the middle of the catheter balloon to the distal end and proximal end of the catheter balloon. Fig. 3 shows an inventive embodiment in form of a coating with a vertical and horizontal concentration gradient. In the middle part of the catheter balloon and on the surface of the coating almost 100% by weight paclitaxel are present while the paclitaxel concentration decreases through the shellac in direction to the surface of the catheter balloon and also in direction to the distal end and the proximal end of the catheter balloon.

Thus the term "vertical concentration gradient" or "radial concentration gradient" as used herein refers to a decreasing concentration of the active agent and especially of paclitaxel from the top of the coating in direction to the balloon surface.

The term "longitudinal concentration gradient" or "horizontal concentration gradient" as used herein refers to a decreasing concentration of the active agent and especially of paclitaxel from the middle or middle part of the balloon surface to the proximal end as well as the distal end of the catheter balloon.

Preferably the coating of the catheter balloon comprises further a base coat of shellac as a first layer under the active agent layer. Also preferred is a catheter balloon, wherein the coating comprises further a top coat of shellac.

The invention is also directed to coating methods of the following type which are especially suited for manufacturing a catheter balloon of the present invention.

One method of the invention for loading or coating dilatable catheter balloons comprises the following steps:

A) providing an uncoated catheter balloon; and
B) providing a solution of an active agent and providing a solution of shellac; and
C) coating the surface of the catheter balloon with the solution of shellac; and
D) applying the solution of the active agent so that two different concentration gradients in vertical and in horizontal direction of the active agent in shellac are induced using a gradient mixer, and subsequently
E) drying the coated catheter balloon

It is preferred that said method comprises further a step D' after step D):

D') applying the solution of shellac again

Of course drying steps can follow after each coating step so a more detailed method reads as follows:

A) providing an uncoated catheter balloon; and
B) providing a solution of an active agent and providing a solution of shellac; and
C) coating the surface of the catheter balloon with the solution of shellac and drying the coated balloon surface; and
D) applying the solution of the active agent and drying the coated balloon surface so that two different concentration gradients in vertical and horizontal direction of the active agent in shellac are induced using a gradient mixer, and subsequently
C) applying the solution of shellac again and
E) drying the coated catheter balloon.

First a catheter balloon and preferably an uncoated catheter balloon or a catheter balloon without any releasable active agent in its surface is provided. Than a solution of an active agent and a second shellac solution each in a suitable solvent such as acetone, ethyl acetate, ethanol, methanol, DMSO, THF, chloroform, methylene chloride or the like is prepared and applied sequentially using conventional coating methods such as spray coating, dip coating etc. in order to obtain after the drying step a solid coating on the surface of the catheter balloon.

It is preferred that step D is carried out in a way that the solution of the active agent penetrates the layer of shellac. Thereby a concentration gradient originates. Preferably the layer of shellac should not soak the solution of the active agent till the surface of the catheter balloon. This means directly on the surface of the catheter balloon stays a base coat or a zone of the ayer of shellac free of the active agent. Hence, preferably the catheter balloon has a base coat which consists of shellac only. The concentration of the active agent increases from zero, or nearly zero, to the maximum with increasing distance from the balloon surface. There could be one zone or layer consisting of pure active agent on top of the coating. For the development of a concentration gradient it is important to use for the solution of the active agent a solvent which does not solve shellac or solves shellac only very bad. One preferred solvent for the active agent and especially for paclitaxel is ethyl acetate. Therefore it is preferred to use two different solvents for shellac and paclitaxel, for example ethanol for shellac and ethyl acetate for paclitaxel. Therefore during a preferred method of the invention for loading or coating catheter balloons the solution of the active agent is prepared using a solvent the solubility for the active agent, preferably paclitaxel, is greater than for shellac.

Solubility is the property of a chemical substance called solute, here the active agent or shellac to dissolve in a solid, liquid, or gaseous solvent to form a homogeneous solution of the solute in the solvent. The solubility of a substance fundamentally depends on the used solvent as well as on temperature and pressure. The extent of the solubility of a substance in a specific solvent is measured as the saturation concentration, where adding more solute does not increase the concentration of the solution.

The drying step E) can be performed at room temperature or at elevated temperatures up to 50°C and at atmospheric pressure or under reduced pressure to high vacuum. The drying step E) may additionally be performed also after step C) and/or after step D), which means a drying step is also possible after the surface of the catheter balloon has been coated firstly with the solution of shellac and after the layer of the active agent has been applied. Thereby the first drying steps are conducted at room temperature and atmospheric pressure, while preferably after the last coating step of the method the drying step is more intensive, i.e. longer or with vacuum or with elevated temperature.

The present invention includes further a method for loading or coating dilatable catheter balloons comprising the following steps:

A) providing an uncoated catheter balloon; and
B*) providing a solution of an active agent, a solution of shellac and providing a solution of the active agent and shellac; and
C) coating the surface of the catheter balloon with the solution of shellac; and
D*) applying the solution of the active agent and the solution of the active agent and shellac using a gradient mixer to induce a vertical and a horizontal concentration gradient of the active agent, and subsequently
C) applying the solution of shellac again and
E) drying the coated catheter balloon.

In step D*) the gradient mixer is filled in the first chamber with a solution of shellac and of the active agent, wherein the active agent has a low concentration and in the second chamber the solution of the active agent in a higher concentration. The mixed solution which comes of the gradient mixer is then sprayed to the balloon using a spray pistol or is pipette to the balloon.

Again the gradient on the balloon has an increasing concentration of the active agent and a decreasing concentration of shellac with increasing distance from the balloon surface towards the top coat. Thereby it is possible that the gradient has different steps where the concentration raises discontinuously but it is especially preferred if the concentration gradient is continuously.

According to the present invention the coating method induces two different concentration gradients in radial (vertical) and longitudinal (horizontal) direction of the active agent in shellac. Thus, not only a vertical concentration gradient of said active agent, but also a vertical and horizontal concentration gradient of said active agent at the same time is applied in the coating by appropriate application of the active agent solution on a pure shellac coating applied first.

In case of a vertical gradient on the balloon, the active agent exhibits an increasing concentration from the balloon surface towards the top of the coating, whereas at the same time a decreasing concentration of shellac with increasing distance from the balloon surface is observed. Preferably, a rather inner layer near the balloon surface is composed of shellac with a low concentration of paclitaxel, while in the same coating a rather outer coating layer near the top of the coating (the part of the coating which comes into direct contact with the vessel wall during dilatation) has a higher concentration of paclitaxel, and thus a lower concentration of shellac.

When two concentration gradients are applied by a coating method of the present invention a horizontal gradient of active agent in the direction of the longitudinal axis of the balloon is created in addition to an above described vertical concentration gradient. Accordingly, the highest concentration of the active agent can be found according to a preferred embodiment in the middle of the coated balloon and the lowest concentration at the proximal and distal end of the balloon. In another preferred embodiment of the present invention a region with the highest concentration of active agent according to the longitudinal axis of the balloon can also be applied towards either the distal or proximal end of the balloon. According to a coating method of the present invention the highest concentration peak in longitudinal direction can be applied at any length between distal and proximal, however preferably in close vicinity or at the middle.

As a further preferred result of the inventive coating method the highest concentration of the active agent is located at the surface of gradient coating in the horizontal middle of the balloon (see also Figure 3). The concentration differences of the gradient can easily be controlled by the concentration difference of the two applied solutions of the active agent and shellac and further by the mixing velocity of said two solutions in a gradient mixer. In a preferred embodiment of the present invention two solutions filled in the first and second chamber of a gradient mixer are on the one hand a solution of an active agent, like paclitaxel, in an appropriate solvent and on the other hand a pure shellac solution. In another preferred embodiment of the present invention a solution of an active agent in a low or high concentration together with shellac and a solution of shellac can be used. Furthermore, in another preferred embodiment a high concentration of the active agent in a shellac solution and a low concentration of said active agent in a shellac

solution can be applied.. Pipetting, spray coating, spattering and brush coating can be used for the inventive coating methods in order to apply of active agent solutions and the shellac solutions.

An inventive coating method can optionally further comprise step F):

F) Sterilization of the paclitaxel and shellac coated catheter balloons.

The sterilization is most preferably performed with ethylene oxide.

The invention is furthermore directed to a catheter balloon comprising a coating with an active agent and shellac, wherein the coating comprises a concentration gradient of the active agent and optionally a base coat of shellac, and a top coat or layer of shellac. It is preferred that the catheter balloon of the invention has a separate top coating of shellac which covers the active agent containing layer.

The term "base coat" as used herein refers to a layer of the coating of a catheter balloon which is immediately on the surface of the catheter balloon. This layer is a first layer which directly overlays the material of the catheter balloon. The term "top layer" or "topcoat" as used herein refers to a layer of the coating free of an active agent which overlays the active agent containing layer.

The term "uncoated" as used herein refers to a catheter balloon with a smooth or structured or roughened surface without any drug coating, i.e. the balloon surface does not comprise a pharmaceutically active agent and especially no anti-proliferative, anti-angiogenic or anti-restenosis drug and no coating containing an anti-proliferative, anti-angiogenic or anti-restenosis drug.

The term "gradient" as used herein refers to is a concentration gradient. This means that in the coating of the catheter balloon according to the invention is a gradual difference in the concentration of the active agent, preferably of paclitaxel, in shellac between two regions. It is preferred that this regions are located radial or vertical to the catheter balloon that the lowest concentration of the active agent, like paclitaxel is directly on the surface of the catheter balloon (on the base material the balloon is made of) and the highest concentration is on top of the coating, which means on the end which comes in contact to the tissue. Exceptions are the embodiments which comprise a top coat of pure paclitaxel. There it is preferred that the highest concentration is on top of the active agent containing layer, which means directly below the top coat. In general a radial or vertical concentration gradient as described below is the preferred embodiment of the present invention. Further preferred is that the catheter balloon of the invention has more than one gradient, which means that there are gradual differences in the concentration of the active agent, preferably of paclitaxel, in shellac between four regions. Thereby the direction of said gradients should differ. It is especially preferred that beside the radial gradient described a longitudinal or horizontal gradient is present in the balloon coating, which means that the longitudinal or horizontal is an additional concentration gradient to the radial gradient. Here the regions are located longitudinal to the catheter balloon, so that for example the lowest concentration of the active agent, like paclitaxel is directly at one or both ends of the catheter balloon (where the balloon ends and the catheter or the catheter tip starts) and the highest concentration is in the middle of the balloon (cf. figure 3).

The concentration gradient may be continuously (linear) or stepwise wherein a continuously or linear concentration change is preferred. It is further preferred that the gradient encompasses the hole depth of coating. This means that the gradient starts directly at the balloon surface and ends on top of the coating. This means that directly at the surface of the catheter balloon the concentration of the active agent, preferably paclitaxel, is 0% in 100% shellac, preferred is 10% in 90% shellac and on top of the coating the concentration of the active agent, preferably paclitaxel, is 100% without shellac. It is further preferred that 75%, even more preferred 80% of the amount of the active agent, preferably paclitaxel is in the upper third of the catheter balloon coating.

Preferred is a catheter balloon, wherein the active agent is an antiproliferative, immunosuppressive, anti-angiogenic, anti-inflammatory, and/or anti-thrombotic agent. It is preferred if the active agent is selected from the group consisting of or comprising:

abciximab, acemetacin, acetylvismione B, aclarubicin, ademetionine, adriamycin, aescin, afromosone, akagerine, aldesleukin, amidorone, aminoglutethimide, amsacrine, anakinra, anastrozole, anemonin, anopterine, antimycotics antithrombotics, apocymarin, argatroban, aristolactam-All, aristolochic acid, ascomycin, asparaginase, aspirin, atorvastatin, auranofin, azathioprine, azithromycin, baccatin, bafilomycin, basiliximab, bendamustine, benzocaine, berberine, betulin, betulinic acid, bilobol, bisparthenolidine, bleomycin, combrestatin, Boswellic acids bruceanol A, B and C, bryophyllin A, busulfan, antithrombin, bivalirudin, cadherins, camptothecin, capecitabine, o-carbamoyl-phenoxyacetic acid, carboplatin, carmustine, celecoxib, cepharanthin, cerivastatin, CETP inhibitors, chlorambucil, chloroquine phosphate, cicutoxin, ciprofloxacin, cisplatin, cladribine, clarithromycin, colchicine, concanamycin, coumadin, C-type natriuretic peptide (CNP), cudraisoflavone A, curcumin, cyclophosphamide, ciclosporin A, cytarabine, dacarbazine, daclizumab, dactinomycin, dapsone, daunorubicin, diclofenac, 1,11-dimethoxycanthin-6-one, docetaxel, doxorubicin, daunamycin, epirubicin, erythromycin, estramustine, etoposide, everolimus, filgrastim, fluroblastin, fluvastatin, fludarabine, fludarabine-5'-dihydrogen phosphate, fluorouracil, folimycin, fosfestrol, gemcitabine, ghalakinoside, ginkgol, ginkgolic acid, glycoside 1a, 4-hydroxyoxycyclo phosphamide, idarubicin, ifosfamide, josamycin, lapachol, lomustine, lovastatin, melphalan, midecamycin, mitoxantrone, nimustine, pitavastatin, pravastatin, procarbazine, mitomycin, methotrexate, mercaptopurine, thioguanine, oxaliplatin, irinotecan, topotecan, hydroxycarbamide, miltefosine, pentostatin, pegaspargase, exemestane, letrozole, formestane, mycophenolate mofetil, β-lapachone, podophyllotoxin, podophyllic acid-2-ethyl hydrazide, molgramostim (rhuGM-CSF), peginterferon α-2b, lenograstim (r-HuG-CSF), macrogol, selectin (cytokine antagonist), cytokinin inhibitors, COX-2 inhibitor, angiopeptin, monoclonal antibodies inhibiting muscle cell proliferation, bFGF antagonists, probucol, prostaglandins, 1-hydroxy-11-methoxycanthin-6-one, scopoletin, NO donors, pentaerythrityl tetranitrate and sydnoimines, S-nitroso derivatives, tamoxifen, staurosporine, β-estradiol, α-estradiol, estriol, estrone, ethinyl estradiol, medroxyprogesterone, estradiol cypionates, estradiol benzoates, tranilast, kamebakaurin and other terpenoids used in cancer therapy, verapamil, tyrosine kinase inhibitors (tyrphostins), paclitaxel and derivatives thereof, 6-α-hydroxy-paclitaxel, taxoteres, mofebutazone, lonazolac, lidocaine, ketoprofen, mefenamic acid, piroxicam, meloxicam, penicillamine, hydroxychloroquine, sodium aurothiomalate, oxaceprol, β-sitosterol, myrtecaine, polidocanol, nonivamide, levomenthol, ellipticine, D-24851 (Calbiochem), colcemid, cytochalasin A-E, indanocine, nocodazole, bacitracin, vitronectin receptor antagonists, azelastine, guanidyl cyclase stimulator, tissue inhibitor of metal proteinase-1 and -2, free nucleic acids, nucleic acids incorporated into virus transmitters, DNA and RNA fragments, plasminogen activator inhibitor 1, plasminogen activator inhibitor 2, antisense oligonucleotides, VEGF inhibitors, IGF-1, active agents from the group of antibiotics, cefadroxil, cefazolin, cefaclor, cefoxitin, tobramycin, gentamicin, penicillins, dicloxacillin, oxacillin, sulfonamides, metronidazole, enoxaparin, heparin, hirudin, PPACK, protamine, prourokinase, streptokinase, warfarin, urokinase, vasodilators, dipyramidole, trapidil, nitroprussides, PDGF antagonists, triazolopyrimidine, seramin, ACE inhibitors, captopril, cilazapril, lisinopril, enalapril, losartan, thioprotease inhibitors, prostacyclin, vapiprost, interferon α, β and γ, histamine antagonists, serotonin blockers, apoptosis inhibitors, apoptosis regulators, halofuginone, nifedipine, tocopherol, tranilast, molsidomine, tea polyphenols, epicatechin gallate, epigallocatechin gallate, leflunomide, etanercept, sulfasalazine, tetracycline, triamcinolone, mutamycin, procainimide, retinoic acid, quinidine, disopyrimide, flecainide, propafenone, sotalol, natural and synthetically obtained steroids such as bryophyllin A, inotodiol, maquiroside A, ghalakinoside, mansonine, strebloside, hydrocortisone, betamethasone, dexamethasone, non-steroidal substances (NSAIDS), fenoprofen, ibuprofen, indomethacin, naproxen, phenylbutazone, antiviral agents, acyclovir, ganciclovir zidovudine, clotrimazole, flucytosine, griseofulvin, ketoconazole, miconazole, nystatin, terbinafine, antiprotozoal agents, chloroquine, mefloquine, quinine, natural terpenoids, hippocaesculin, barringtogenol-C21-angelate, 14-dehydroagrostistachin, agroskerin, agrostistachin, 17-hydroxyagrostistachin, ovatodiolids, 4,7-oxycycloanisomelic acid baccharinoids B1, B2, B3 and B7, tubeimoside, bruceantinoside C, yadanziosides N and P, isodeoxyelephantopin, tomenphantopin A and B, coronarin A,B C and D, ursolic acid, hyptatic acid A, iso-iridogermanal, maytenfoliol, effusantin A, excisanin A and B, longikaurin B, sculponeatin C, kamebaunin, leukamenin A and B, 13,18-dehydro-6-alpha-senecioyloxychaparrin, taxamairin A and B, regenilol, triptolide, cymarin, hydroxyanopterine, protoanemonin, cheliburin chloride, sinococuline A and B, dihydronitidine, nitidine chloride, 12-β-hydroxypregnadien-3,20-dione, helenalin, indicine, indicine-N-oxide, lasiocarpine, inotodiol, podophyllotoxin, justicidin A and B, larreatin, malloterin, mallotochromanol, isobutyrylmallotochromanol, marchantin A, maytansin, lycoridicin, margetine, pancratistatin, liriodenine, oxoushinsunine, periplocoside A, deoxypsorospermin, psychorubin, ricin A, sanguinarine, manwu wheat acid, methylsorbifolin, chromones of spathelia, stizophyllin, dihydrousambaraensine, hydroxyusambarine, strychnopentamine, strychnophylline, usambarine, usambarensine, liriodenine, daphnoretin, lariciresinol, methoxylariciresinol, syringaresinol, sirolimus (rapamycin), biolimus A9, pimecrolimus, everolimus, zotarolimus, tacrolimus, fasudil, epothilones, somatostatin, roxithromycin, troleandomycin, simvastatin, rosuvastatin, vinblastine, vincristine, vindesine, teniposide, vinorelbine, trofosfamide, treosulfan, temozolomide, thiotepa, tretinoin, spiramycin, umbelliferone, desacetylvismione A, vismione A and B, zeorin.

Basically any active agent as well as combination of active agents can be used, wherein, however, paclitaxel, taxanes, docetaxel as well as rapamycin, biolimus A9, pimecrolimus, everolimus, zotarolimus, tacrolimus, fasudil and epothilones are preferred and particularly preferred are paclitaxel and rapamycin. The use of paclitaxel is preferred. Thus all ranges and values given herein and all embodiments disclosed herein are especially in regard to paclitaxel and should be first of all interpreted in this way.

Therefore the present invention relates to catheter balloon comprising a coating with paclitaxel and shellac, wherein the coating comprises a vertical and horizontal concentration gradient of the active agent. Paclitaxel is commercially available from several suppliers. Paclitaxel is known under the trademark name of Taxol® and is also designated with various synonymous names such as:

BMS 181339-01, BMS-181339, BMS-181339-01, Capxol, DRG-0190, DTS-301, Ebetaxel, Genaxol, Genexol, Genexol-PM, HSDB 6839, Intaxel, KBio2_002509, KBio2_005077, KBio2_007645, KBio3_002987, KBioGR_002509, KBioSS_002517, LipoPac, MBT 0206, MPI-5018,

Nanotaxel, NCI60_000601, Nova-12005, NSC 125973, NSC-125973, NSC125973, Onxol, Pacligel, Paxceed, Paxene, Paxoral, Plaxicel, QW 8184, SDP-013, TA1, Tax-11-en-9-on, TaxAlbin, Taxol A, Xorane or Yewtaxan.

Its chemical structure is as follows:

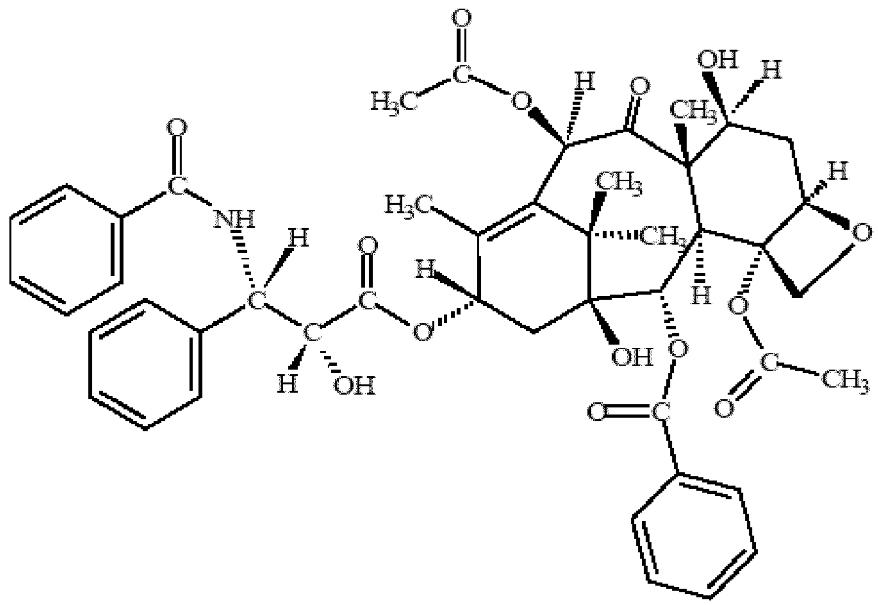

IUPAC nomenclature is as follows: [2aR-[2a,4,4a,6,9(R*,S*),11,12,12a,12b]]-(benzoylamino)-hydroxybenzene propionic acid 6,12b-bis-(acetyloxy)-12-(benzoyloxy)-2a-3, 4, 4a, 5, 6, 9, 10, 11, 12, 12a, 12b-dodecahydro-4,11-dihydroxy-4a,8,13,13-tetramethyl-5-oxo-7,11-methano-1H-cyclodeca[3,4]benz[1,2-b]oxet-9-yl ester).

Paclitaxel is highly soluble in dimethyl sulfoxide (DMSO) and methanol as well as in anhydrous ethanol, but is comparatively poorly soluble in water. Paclitaxel is especially stable at a pH between 3 and 5 and can be stored for long periods, whereas it is comparatively instable at alkaline pH.

Dimethyl sulfoxide (DMSO), acetone, ethyl acetate, ethanol and methanol are used as a solvent for paclitaxel.

As a very prosperous active agent for the same purpose of restenosis prophylaxis rapamycin (syn. sirolimus) a hydrophilic macrolid antibiotic appears. This active agent is especially utilized in transplantation medicine as immunosuppressive, wherein contrary to other immunosuppressive active agents rapamycin also inhibits tumour formation. As after transplantation an increased risk of tumor formation exists for the patient the administration of rapamycin is advantageous because other immunosuppressives such as cyclosporin A can even promote tumor formation as is known.

Its chemical structure is as follows:

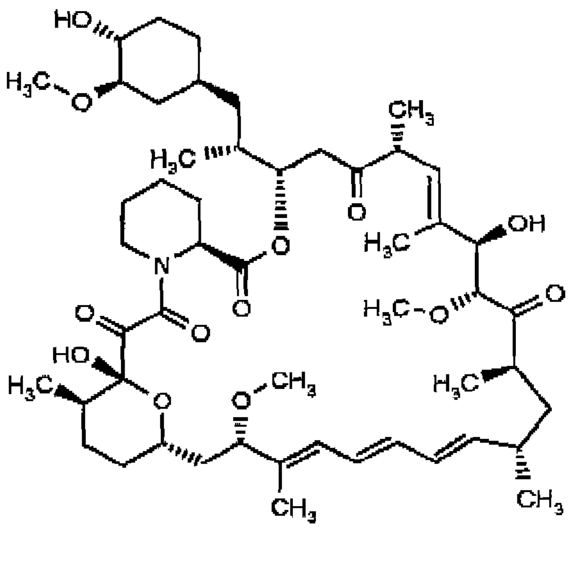

IUPAC name:

[3S-[3R*[E(1S*,3S*,4S*)1,4S*,5R*,8S*,9E,12R*,14R*,15*,16R*,18S*,19S*,26aR*]]-5,6,8,11,12,13,14,15,16,17,18,19,24,25,26,26a-hexadecahydro-5,19-dihydroxy-3-[2-(4-hydroxy-3-methoxycyclohexyl)-1-methy[ethenyl]-14,16-dimethoxy-4,10,12,18-tetramethyl-8-(2-propenyl)-15,19-epoxy-3H-pyrido[2,1-c][1,4]-oxaazacyclotricasine-1,7,20,21(4H,23H)-tetron monohydrate.

Rapamycin's mechanism of action is not yet known in detail but it is attributed especially to the complex formation with the protein mTOR (mammalian target of rapamycin) a phosphatidylinositol-3 kinase of 282kD. As mTOR is responsible for a series of cytokin-mediated signal transduction paths i.a, also for signal paths which are necessary for cell division besides the immunosuppressive effect it has also antiphlogistic, antiproliferative and even antimycotic properties.

Proliferation is interrupted in the late G1 phase by stopping the ribosomal protein synthesis. Compared to other antiproliferative active agents rapamycin's mechanism of action can be pointed out as special likewise paclitaxel but which is strongly hydrophobic. Moreover, the immunosuppressive and antiphlogistic effects as described above are more than advantageous because also the extent of inflammatory reactions and of the total immune response as their premature control after stent implantation is decisive for the further success.

Thus, rapamycin has all of the necessary conditions for the utilization against stenoses and restenoses. Rapamycin's limited shelf life on or in an implant is to be mentioned as an additional advantage in comparison to paclitaxel because necessarily the active agent has to be effective in the first decisive weeks after stent implantation. Consequently, the endothelial cell layer which is important for the completion of a healthy healing process can completely grow over the stent and integrate it into the vessel wall.

The same mechanism of action can be found for the known derivatives of rapamycin (biolimus, everolimus) as the modification is on the molecule's functional groups which are irrelevant for the binding region of mTOR. In different clinical studies (RAVEL, SIRIUS, SIROCCO) rapamycin has shown - contrary to other active agents such as dexamethason, tacrolimus, batimastat - that in comparison to the strongly hydrophobic paclitaxel despite of different physical properties it is more than suitable for combating restenosis.

It was surprisingly found that an active agent - shellac - coating, comprising a gradient of the active agent, like paclitaxel, in a matrix of shellac is therapeutically highly useful in keeping blood vessels open, in reducing the late lumen loss and in reducing restenosis.

An active agent, especially paclitaxel, itself is no warrant for an optimal prophylaxis of restenosis. The active agent, especially paclitaxel -eluting catheter balloon has to meet the requirements in its entirety. Besides the determination of dosing the active agent elution has to be effective during the short time of dilatation (around 30 sec.). The active agent elution as well as the rate of sirolimus elution do not depend only on the physical and chemical properties of the active agent but depends also on the properties of the utilized matrix and the interactions of the matrix and the active agent.

Unlike other similar balloon coatings, no 1: 1 mix of the active agent with the matrix is present in the coating of the drug delivery balloon but a gradient. This leads to a reduced transfer of the matrix substance shellac to the vessel wall with unchanged amount of the active agent.

The inventive balloon coating ensures that the at least one an antiproliferative, immunosuppressive, anti-angiogenic, anti-inflammatory, and/or anti-thrombotic agent, preferably paclitaxel, is released directly and clearly to the vessel wall during balloon inflation because the active agent in the coating is approximate to the surface of the coating. The active agent is immediately and clearly purer and highly concentrated when brought into contact to the vessel wall, i.e. without large proportion of shellac.

The clinical benefit is the purer drug delivery, leading to a significantly higher bioavailability in arterial tissues. This higher drug concentration in the tissue of the vessel wall provides increased effectiveness against migration and proliferation of vascular muscle cells towards the lumen of the artery at the site of the treated stenosis. Neointimal hyperplasia is more effectively suppressed.

Materials used for the balloon catheter are such materials as listed further below, wherein the following polymers are particularly preferred: polyamides, block copolymers of polyamide, polyether and polyester, polyurethanes, polyesters and polyolefins.

The catheter balloon is dilatable or expandable and is most preferably an angioplasty catheter balloon which could be used without crimped stent or with a crimpled stent. As stent, all kinds of common stents, such as self-expandable stents, not self-expandable stents, metal stents, polymer stents, biodegradable stents, bifurcation stents, uncoated (bare) stents, polymer coated stents, drug release coated stents, stents with a pure active agent coating etc. can be used.

Moreover, the stent can be crimped on the catheter balloon before the inventive coating procedure is carried out so that catheter balloon and stent are coated together with a shellac-active agent coating. However, it is preferred to use the coated catheter balloon of the present invention without stent.

The provided catheter balloon is normally a multifold catheter balloon which will also be coated under or within the folds. Moreover it is possible to selectively coat or fill the folds. The coating within or under the folds has the advantage

that during insertion of the catheter balloon the coating and thus the paclitaxel is protected against being washed off by the blood stream.

Furthermore, the catheter balloon can be coated in its expanded (inflated) or deflated state. Any commercially available dilatable catheter balloon may be used as catheter balloon. Preferably, so called multifold balloons are used, as described for example in the international patent application WO 94/23787 A1 by David H. Rammler, Labintelligence, USA; or the international patent application WO 03/059430 A1 by Scimed Life Sciences, Inc., USA; or the international patent application WO 2004/028582 A1 by Prof. Dr. Ulrich Speck or the European Patent No. EP 0519063 B1 by Medtronic Inc., USA.

Such balloons are provided with folds or wings forming essentially closed cavities when the balloon is in its compressed state but bending outward during dilatation and being capable of releasing substances contained in the folds or respectively of pressing said substances against the vessel wall.

Such balloons are advantageous since the substances enclosed in the folds or respectively paclitaxel enclosed in the folds are protected from being detached too soon during the insertion of the catheter.

Regardless of the source of shellac, all kinds of shellac types obtained from various locations or from different insects were able to achieve the inventive results so that any kind or sort of shellac can be used in the present invention. Thus there are no limitations in regards of shellac.

Shellac is a natural resin produced from the glandular secretion of a number of species of lac-producing insects. Lac insects belong to the order of Hemiptera, superfamily Coccoidea such as Metatachardia, Laccifer, Tachordiella, and others, however, members of two families - Lacciferidae and Tachardinidae are more prominent in lac secretion. The one that is commercially cultured is Kerria lacca, which is also known by such synonyms as Laccifer lacca Ker, Tachardia lacca, and Carteria lacca. Kerria lacca is an Indian scale insect, which infests branches of numerous trees from the East Indies, such as Butea frondos Rosch, Acacia arabica Willd and Ficus religiosa Linn. Shellac is the only commercially used natural resin of animal origin and is quite different from all other natural resins. More recently, as a new awareness about the environments and the toxicity of chemical raw-material is noticeable everywhere, shellac or shellac modified resin are gaining importance due to their interesting and unique characteristics. Broken branches are sold as stick lac and, after grounding and washing with water to eliminate wood and red pigments (lac dye), seed lac is obtained. Purification of seed lac gives the more homogeneous product known as shellac. Raw material shellac consists of 70-80% resin, 4-8% dye, 6-7% hard and high gloss finished wax, 3% water, up to 9% vegetable and animal impurities and aroma substances. Shellac resin is a complicated mixture of aliphatic (60%) and sesquiterpenoid acids (32%) and their esters. Sesquiterpenoid acids are jalaric and laccijalaric acids (structure I and II) and aliphatic acids are aleuritic (III) and butolic acid.

A possibility for chemical description of resin molecule is a structure model where in each case 4 molecules jalaric or laccijalaric acid and aleuritic acid are connected by ester bonding alternately.

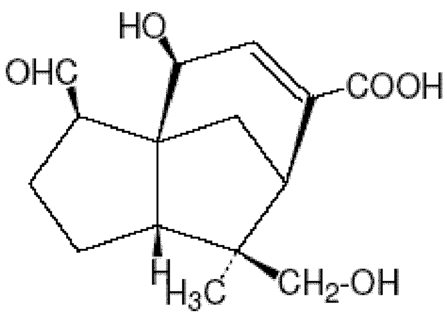

jalaric acid (I)

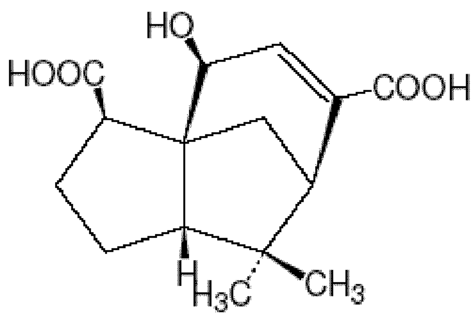

laccijalaric acid (II)

Its chemical composition is almost constant, although the amount of some components changes depending on the nature of host trees on which the insects grows. By Cannizzaro-type disproportionation under alkaline hydrolysis will be synthesized from these acids shellolic acid (IV) and derivate compounds. Purified shellac consists of two main components. These components are 9,10,16-trihydroxypalmitic acid (aleuritic acid) CAS [53-387-9] and shellolic acid (IV).

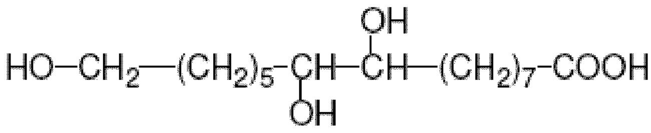

aleuritic acid (III)

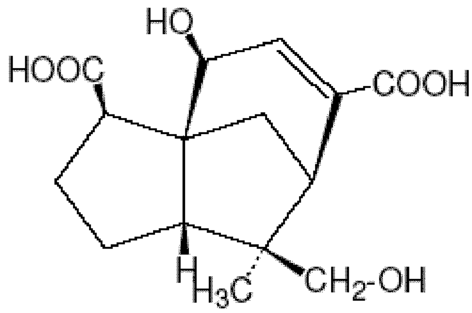

shellolic acid (IV)

A modification with other natural or synthetic resins or co-polymerization with various monomers is possible to cross link shellac, modified shellac resins and shellac copolymers with urea, melamine, formaldehyde, isocyanides, other chemical processes like polymerization, hydroxylation, extrication, etc. are possible.

Followings are the commercial grades of shellac:

- Seedlac
- Hand Made Shellac
- Machine Made Shellac
- Dewaxed Shellac
- Dewaxed Bleached Shellac
- Aleuritic Acid

Major Properties of shellac are:

- Shellac is a hard natural resin
- Shellac has a good resistance against solvent
- Shellac based on hydrocarbons
- Shellac is non toxic
- Shellac is thermoplastic
- Shellac is physiologically harmless
- Shellac is approved for various applications in the food industry.
- Shellac is not UV-resistant
- Shellac is soluble in lower alcohol's
- Shellac has excellent dielectric properties high dielectric strength, low dielectric constant, good tracking resistance etc.
- Shellac has a low melting point (65-85°C).
- Shellac is water soluble in water-alkaline solutions
- Coatings do not change their electric properties under UV-radiation.
- Shellac has excellent film forming properties.
- Shellac has low thermal conductivity and a low coefficient of expansion forms smooth, high gloss films and surfaces.
- Shellac coating has excellent adhesion to many coatings and can be polished.
- Shellac can be cross linked to modify other natural/synthetic resins Examples for industrial uses:
- Coating of pills & tablets
- Coating Fruits
- Cosmetics
- French polish surface coating, sealer
- Optical frames

The catheter balloons according to the invention were coated with different commercial grades of shellac as well as with varying batches, which differed in the Lac insects, and host tree types used as well as in the time of harvest. There were no differences in release of the active agents observable in various coated catheter balloons.

Generally, an amount of 0.1 μg to 30 μg of paclitaxel per $mm^2$ of the surface of the balloon catheter to be coated can be applied onto the surface of the balloon catheter, while an amount of 0.5 μg/$mm^2$ to 12 μg/$mm^2$ of paclitaxel is sufficient in order to achieve the desired effect on restenosis prophylaxis. The surface load of the active agent, and preferably of paclitaxel, on the catheter balloon is between 0.1 μg/$mm^2$ and 30 μg/$mm^2$. Preferably the amount of the

active agent present on the coated balloon surface is between 1 μg/mm² and 12 μg/mm² balloon surface, more preferably between 2μg/mm² and 10 μg/mm² and most preferably between 2.5 μg and 5 μg active agent per mm² balloon surface (μg/mm²).

Preferred is also a total amount of 10 to 1000 μg of an active agent, preferably paclitaxel, per catheter balloon and most preferably 20 to 400 μg per catheter balloon.

The surface load of shellac on the catheter balloon is between 1 μg/mm² and 12 μg/mm². Preferably the amount of shellac present in the base coat on the coated balloon surface is between 0.5 μg/mm² and 5 μg/mm² balloon surface, more preferably between 1 μg/mm² and 4 μg/mm² and most preferably between 1.5 μg/mm² and 2 μg shellac per mm² balloon surface (μg/mm²). Preferably the amount of shellac present in the top coat on the coated balloon surface is between 0.1 μg/mm² and 3 μg/mm² balloon surface, more preferably between 0.5 μg/mm² and 2 μg/mm² and most preferably between 0.8 μg and 1.5 μg shellac per mm² balloon surface (μg/mm²).

The surface of the catheter balloon may be textured, smooth, rough, harsh, provided with cavities or provided with channels open towards the outside of the balloon.

In the case, a textured surface of the catheter balloon is desired, the surface of the catheter balloon can be textured mechanically, chemically, electronically and/or by means of radiation to allow for an improved adhesion of paclitaxel and to assist the precipitation or crystallization of the paclitaxel.

It is of importance to avoid all damage to the catheter balloons while the balloon surface is textured and to ensure that their capability to expand is not disadvantageously affected. Thus, the methods for micro texturing the balloon surface must not lead to the formation of holes, micropores or fissures in the balloon material. Ideally, only the outer surface of the balloon, i.e. to a maximum depth of 1 μm, is textured.

The content of the active agent in the active agent containing solution is between 1 μg to 1 mg of the active agent per ml solution, preferably between 10 μg to 500 μg of the active agent per 1 ml solution, more preferably between 30 μg to 300 μg of the active agent per 1 ml solution, and most preferably between 50 μg to 100 μg of the active agent per 1 ml solution.

According to the invention, the catheter balloon does not have to be completely coated. Partial coating of the catheter balloon or partial loading of certain texture elements onto the surface of the catheter balloon may be sufficient. A special catheter balloon including micro-needles or micro-pores or micro-chambers is disclosed in the international patent application no. WO 02/043796 A2 issued to Scimed Life Systems, Inc., USA, wherein inflatable and textured areas are present on the balloon surface. In said embodiment, loading or inflating certain portions of the balloon surface would be sufficient to achieve the desired therapeutic success, wherein it is also possible, evidently, that the whole surface is coated.

A especially preferred embodiment of the present invention is directed to a catheter balloon coated with shellac and paclitaxel with a concentration gradient of paclitaxel in direction to the balloon surface so that on top of the coating almost 100% by weight paclitaxel is present and directly on the surface of the balloon almost 100% by weight shellac is present while the concentration of paclitaxel in shellac decreases from 100% by weight from the top of the coating to 0% by weight directly on the surface of the balloon.

In addition to this vertical concentration gradient which is perpendicular to the longitudinal axis of the catheter balloon, a horizontal concentration gradient is present.

Such a horizontal concentration gradient means that in the middle of the catheter balloon the highest concentration of paclitaxel is present and this concentration of paclitaxel will decrease in proximal and also in distal direction so that the lowest paclitaxel concentration is present at the proximal and distal ends of the catheter balloon.

Referring the Fig. 3 is becomes obvious how a coating with a radial (vertical) and a longitudinal (horizontal) concentration gradient looks like. On top and in the middle of the catheter balloon almost 100% by weight paclitaxel and 0% by weight shellac is present while immediately on the balloon surface distal and proximal of the catheter balloon 0% by weight paclitaxel and 100% by weight shellac is present. The paclitaxel concentration decreases from the top of the coating through the coating to the surface of the catheter balloon as well as from the middle part of the catheter balloon to the distal and proximal ends of the catheter balloon. This special kind of coating ensures paclitaxel application in high doses exactly in the center of the stenosis or diseased vessel area and application of lower paclitaxel amounts proximal and distal of the stenosis or diseased vessel area. Thus paclitaxel application is perfectly adjusted to the stenosis area or diseased vessel area and high doses of active agent are administered where needed while decreasing doses are administered in the side regions of the stenosis area or diseased vessel area.

Since the active agent-shellac coating is hard to characterize, the present invention relates also to coated catheter balloons obtained according to the inventive coating methods disclosed herein as well as to balloon catheter and dilatation catheter comprising said active agent-shellac coated catheter balloon or generally a catheter balloon according to the invention.

Such catheters or catheter balloons which are coated according to the invention are preferably used for treating constricted vessel segments, particularly of blood vessels and for the treatment and prophylaxis of stenosis, restenosis, arteriosclerosis and fibrotic vessel constriction.

Catheter balloons which are coated according to the invention are preferably suited for the treatment and prophylaxis of in-stent restenosis, i.e. a reoccurring vessel constriction within an already implanted stent. Furthermore, the catheter balloons coated according to the invention are particularly suited for the treatment of small vessels, preferably such vessels having a vessel diameter of less than 2.25 mm.

The catheter balloons coated according to the invention are preferably used in the cardiovascular area, but the catheter balloons coated according to the invention are also suited for the treatment of peripheral blood vessels, vessel constrictions of biliary tracts, esophagus, urinary tracts, pancreas, renal tracts, pulmonary tracts, trachea, small intestine and large intestine.

Furthermore, a second active agent may be added to the active agent containing solution. Said further active agent can be selected from the following group comprising or consisting of:

abciximab, acemetacin, acetylvismione B, aclarubicin, ademetionine, adriamycin, aescin, afromosone, akagerine, aldesleukin, amidorone, aminoglutethimide, amsacrine, anakinra, anastrozole, anemonin, anopterine, antimycotics antithrombotics, apocymarin, argatroban, aristolactam-AII, aristolochic acid, ascomycin, asparaginase, aspirin, atorvastatin, auranofin, azathioprine, azithromycin, baccatin, bafilomycin, basiliximab, bendamustine, benzocaine, berberine, betulin, betulinic acid, bilobol, bisparthenolidine, bleomycin, combrestatin, Boswellic acids , bruceanol A, B and C, bryophyllin A, busulfan, antithrombin, bivalirudin, cadherins, camptothecin, capecitabine, o-carbamoyl-phenoxyacetic acid, carboplatin, carmustine, celecoxib, cepharanthin, cerivastatin, CETP inhibitors, chlorambucil, chloroquine phosphate, cicutoxin, ciprofloxacin, cisplatin, cladribine, clarithromycin, colchicine, concanamycin, coumadin, C-type natriuretic peptide (CNP), cudraisoflavone A, curcumin, cyclophosphamide, ciclosporin A, cytarabine, dacarbazine, daclizumab, dactinomycin, dapsone, daunorubicin, diclofenac, 1,11-dimethoxycanthin-6-one, docetaxel, doxorubicin, daunamycin, epirubicin, erythromycin, estramustine, etoposide, everolimus, filgrastim, fluroblastin, fluvastatin, fludarabine, fludarabine-5'-dihydrogen phosphate, fluorouracil, folimycin, fosfestrol, gemcitabine, ghalakinoside, ginkgol, ginkgolic acid, glycoside 1a, 4-hydroxyoxycyclo phosphamide, idarubicin, ifosfamide, josamycin, lapachol, lomustine, lovastatin, melphalan, midecamycin, mitoxantrone, nimustine, pitavastatin, pravastatin, procarbazine, mitomycin, methotrexate, mercaptopurine, thioguanine, oxaliplatin, irinotecan, topotecan, hydroxycarbamide, miltefosine, pentostatin, pegaspargase, exemestane, letrozole, formestane, mycophenolate mofetil, $\beta$-lapachone, podophyllotoxin, podophyllic acid-2-ethyl hydrazide, molgramostim (rhuGM-CSF), peginterferon $\alpha$-2b, lenograstim (r-HuG-CSF), macrogol, selectin (cytokine antagonist), cytokinin inhibitors, COX-2 inhibitor, angiopeptin, monoclonal antibodies inhibiting muscle cell proliferation, bFGF antagonists, probucol, prostaglandins, 1-hydroxy-11-methoxycanthin-6-one, scopoletin, NO donors, pentaerythrityl tetranitrate and sydnoimines, S-nitroso derivatives, tamoxifen, staurosporine, $\beta$-estradiol, $\alpha$-estradiol, estriol, estrone, ethinyl estradiol, medroxyprogesterone, estradiol cypionates, estradiol benzoates, tranilast, kamebakaurin and other terpenoids used in cancer therapy, verapamil, tyrosine kinase inhibitors (tyrphostins), paclitaxel , 6-$\alpha$-hydroxy-paclitaxel, taxoteres, mofebutazone, lonazolac, lidocaine, ketoprofen, mefenamic acid, piroxicam, meloxicam, penicillamine, hydroxychloroquine, sodium aurothiomalate, oxaceprol, $\beta$-sitosterol, myrtecaine, polidocanol, nonivamide, levomenthol, ellipticine, D-24851 (Calbiochem), colcemid, cytochalasin A-E, indanocine, nocodazole, bacitracin, vitronectin receptor antagonists, azelastine, guanidyl cyclase stimulator, tissue inhibitor of metal proteinase-1 and -2, free nucleic acids, nucleic acids incorporated into virus transmitters, DNA and RNA fragments, plasminogen activator inhibitor 1, plasminogen activator inhibitor 2, antisense oligonucleotides, VEGF inhibitors, IGF-1, active agents from the group of antibiotics, cefadroxil, cefazolin, cefaclor, cefoxitin, tobramycin, gentamicin, penicillins, dicloxacillin, oxacillin, sulfonamides, metronidazole, enoxaparin, heparin, hirudin, PPACK, protamine, prourokinase, streptokinase, warfarin, urokinase, vasodilators, dipyramidole, trapidil, nitroprussides, PDGF antagonists, triazolopyrimidine, seramin, ACE inhibitors, captopril, cilazapril, lisinopril, enalapril, losartan, thioprotease inhibitors, prostacyclin, vapiprost, interferon $\alpha$, $\beta$ and $\gamma$, histamine antagonists, serotonin blockers, apoptosis inhibitors, apoptosis regulators, halofuginone, nifedipine, tocopherol, tranilast, molsidomine, tea polyphenols, epicatechin gallate, epigallocatechin gallate, leflunomide, etanercept, sulfasalazine, tetracycline, triamcinolone, mutamycin, procainimide, retinoic acid, quinidine, disopyrimide, flecainide, propafenone, sotalol, natural and synthetically obtained steroids such as bryophyllin A, inotodiol, maquiroside A, ghalakinoside, mansonine, strebloside, hydrocortisone, betamethasone, dexamethasone, non-steroidal substances (NSAIDS), fenoprofen, ibuprofen, indomethacin, naproxen, phenylbutazone, antiviral agents, acyclovir, ganciclovir zidovudine, clotrimazole, flucytosine, griseofulvin, ketoconazole, miconazole, nystatin, terbinafine, antiprotozoal agents, chloroquine, mefloquine, quinine, natural terpenoids, hippocaesculin, barringtogenol-C21-angelate, 14-dehydroagrostistachin, agroskerin, agrostistachin, 17-hydroxyagrostistachin, ovatodiolids, 4,7-oxycycloanisomelic acid baccharinoids B1, B2, B3 and B7, tubeimoside, bruceantinoside C, yadanziosides N and P, isodeoxyelephantopin, tomenphantopin A and B, coronarin A,B C and D, ursolic acid, hyptatic acid A, iso-iridogermanal, maytenfoliol, effusantin A, excisanin A and B, longikaurin B, sculponeatin C, kamebaunin, leukamenin A and B, 13,18-dehydro-6-alpha-senecioyloxychaparrin, taxamairin A and B, regenilol, triptolide, cymarin, hydroxyanopterine, protoanemonin, cheliburin chloride, sinococuline A and B, dihydronitidine, nitidine chloride, 12-$\beta$-hydroxypregnadien-3,20-dione,

helenalin, indicine, indicine-N-oxide, lasiocarpine, inotodiol, podophyllotoxin, justicidin A and B, larreatin, malloterin, mallotochromanol, isobutyrylmallotochromanol, marchantin A, maytansin, lycoridicin, margetine, pancratistatin, liriodenine, oxoushinsunine, periplocoside A, deoxypsorospermin, psychorubin, ricin A, sanguinarine, manwu wheat acid, methylsorbifolin, chromones of spathelia, stizophyllin, dihydrousambaraensine, hydroxyusambarine, strychnopentamine, strychnophylline, usambarine, usambarensine, liriodenine, daphnoretin, lariciresinol, methoxylariciresinol, syringaresinol, sirolimus (rapamycin), biolimus A9, pimecrolimus, everolimus, zotarolimus, tacrolimus, fasudil, epothilones, somatostatin, roxithromycin, troleandomycin, simvastatin, rosuvastatin, vinblastine, vincristine, vindesine, teniposide, vinorelbine, trofosfamide, treosulfan, temozolomide, thiotepa, tretinoin, spiramycin, umbelliferone, desacetylvismione A, vismione A and B, zeorin.

Due to the inventive coating method, the active agent-shellac composite dried at the surface of the catheter balloon has a special consistence, which is hard to characterize but seems to be essential for the transfer to the cell wall and the incorporation, especially into the smooth muscle cells.

The following examples illustrate potential embodiments of the invention without limiting the scope of the invention to said precise examples.

**Description of Figures:**

Figure 1: Intramural Paclitaxel concentration [μg/g]

Figure 2: Vessel Intramural percentage of paclitaxel loading dose

Figure 3: Graphical description a gradient paclitaxel coating on a catheter balloon having a radial (vertical) and a longitudinal (horizontal) concentration gradient

**Examples**

Reference-example 1:

Coating of a catheter balloon with paclitaxel and shellac Firstly, 120 mg paclitaxel are solved in 800 μL ethanol and 5 g shellac are solved in 100 mL ethanol stirring for 24 h at room temperature.

The shellac solution is applied to the surface of a fold balloon which is rotatably mounted by a pipetting device. Then the fold balloon is dried under slow rotation at room temperature. The base coat contained 2 $\mu g/mm^2$ shellac on the balloon surface.

The paclitaxel solution is then sprayed on the balloon catheter with the base coat in a way that 3.0 $\mu g/mm^2$ paclitaxel are applied. Then the balloon is dried without rotation at room temperature. Finally, the shellac solution is applied as a separate topcoat by a pipetting device on the active agent layer. 1$\mu g/mm^2$ shellac is applied. Subsequently, the catheter balloon is thoroughly dried for 30 minutes at 50 °C. The presence of a stent or drug-eluting stent crimped on the balloon does not interfere with the coating process.

Reference-example 2:

Coating of a catheter balloon with rapamycin and shellac A commercially available dilatation catheter with expandable balloon made of a polyamide is provided. The balloon surface is textured but without channels or cavities.

A solution of 4 g of shellac in 50 mL of ethanol is prepared and applied onto the horizontal area of the surface of the catheter balloon by brushing. A solution of 140 μg of rapamycin in 2.0 mL of ethanol is prepared and the catheter balloon is immersed into said solution. A topcoat containing 1.5 $\mu g/mm^2$ shellac is applied by brushing. Subsequently, the catheter balloon is thoroughly dried and sterilized with ethylene oxide.

Reference-example 3:

Coating of a catheter balloon with paclitaxel, α-linolenic acid and shellac A balloon of a balloon catheter suitable for expansion vessel dilatation is degreased with acetone and ethanol in an ultrasonic bath for 10 minutes and the balloon catheter is then dried at 100 ° C. A solution of 4 g of shellac in 50 mL of ethanol is prepared and applied onto the horizontal area of the surface of the catheter balloon by brushing. A spray solution of α-linolenic acid with 0.25 weight percent in ethanol is prepared and sprayed with a spray gun evenly over the surface of the balloon of an evenly rotating balloon

catheter. 120 mg paclitaxel are solved in 1 mL ethyl acetate. This solution is applied to the catheter balloon by spraying. The shellac solution is applied again as a thin topcoat. The coated catheter balloon is dried within 13 hours at 70 °C.

Reference-example 4:

Coating of a catheter balloon with paclitaxel and shellac using a gradient mixer Firstly, 120 mg paclitaxel are solved in 800 μL ethanol and 5 g shellac are solved in 100 mL ethanol stirring for 24 h at room temperature. After this 100 μL of the solution of paclitaxel is mixed with 900 μL of the shellac solution.

The pure shellac solution is applied to the surface of a fold balloon which is rotatably mounted by a pipetting device. Then the fold balloon is dried under slow rotation at room temperature. The base coat contained 1 $\mu g/mm^2$ shellac on the balloon surface.

The solution containing paclitaxel and shellac is poured in the first chamber of a gradient mixer and the pure paclitaxel solution is poured in the second, posterior chamber. The outlet of the gradient mixer is connected to a spray gun. The solution out of the gradient mixer is then sprayed on the balloon catheter with the base coat in a way that increasing paclitaxel concentration is applied. A total of 3.0 $\mu g/mm^2$ paclitaxel is applied. Then the balloon is dried under slow rotation at room temperature. Finally, the shellac solution is applied as a separate topcoat by a pipetting device on the active agent layer. 1 $\mu g/mm^2$ shellac is applied. Subsequently, the catheter balloon is thoroughly dried for 30 minutes at 50 °C. The presence of a stent or drug-eluting stent crimped on the balloon does not interfere with the coating process.

Reference-example 5:

Coating of a catheter balloon with paclitaxel and shellac using a gradient mixer Firstly, 120 mg paclitaxel are solved in 800 μL ethanol and 5 g shellac are solved in 100 mL ethanol stirring for 24 h at room temperature. After this 100 μL of the solution of paclitaxel is mixed with 900 μL of the shellac solution.

The pure shellac solution is applied to the surface of a fold balloon which is rotatably mounted by a spraying device. Then the fold balloon is dried under slow rotation at room temperature. The base coat contained 1 $\mu g/mm^2$ shellac on the balloon surface.

The solution containing paclitaxel and shellac is poured in the first chamber of a gradient mixer and the pure paclitaxel solution is poured in the second, posterior chamber. The outlet of the gradient mixer is connected to a spray gun. The solution out of the gradient mixer is then sprayed on the balloon catheter with the base coat in a way that increasing paclitaxel concentration is applied. A total of 3.0 $\mu g/mm^2$ paclitaxel is applied. Then the balloon is dried under slow rotation at room temperature.

Reference-example 6:

Coating of a catheter balloon with paclitaxel and shellac by a pipetting method using a gradient mixer Firstly, 120 mg paclitaxel are solved in 800 μL ethanol and 5 g shellac are solved in 100 mL ethanol stirring for 24 h at room temperature. After this 100 μL of the solution of paclitaxel is mixed with 900 μL of the shellac solution.

The solution containing paclitaxel and shellac is poured in the first chamber of a gradient mixer and the pure paclitaxel solution is poured in the second, posterior chamber. The outlet of the gradient mixer is connected to a pipetting device. The solution out of the gradient mixer is then pipetted on the balloon catheter with the base coat in a way that increasing paclitaxel concentration is applied. A total of 3.0 $\mu g/mm^2$ paclitaxel is applied. Then the balloon is dried under slow rotation at room temperature. Finally, the shellac solution is applied as a separate topcoat by a pipetting device on the active agent layer. 1 $\mu g/mm^2$ shellac is applied. Subsequently, the catheter balloon is thoroughly dried for 30 minutes at 50 °C. The presence of a stent or drug-eluting stent crimped on the balloon does not interfere with the coating process.

Reference-example 7:

Coating of a catheter balloon with paclitaxel and shellac using a gradient mixer Firstly, 120 mg paclitaxel are solved in 800 μL ethyl acetate and 5 g shellac are solved in 100 mL ethanol stirring for 24 h at room temperature. After this 100 μL of the solution of paclitaxel is diluted with 900 μL of ethyl acetate.

The pure shellac solution is applied to the surface of a fold balloon which is rotatably mounted by a pipetting device. Then the fold balloon is dried under slow rotation at room temperature. The base coat contained 1 $\mu g/mm^2$ shellac on the balloon surface.

A paclitaxel solution in a low concentration is poured in the first chamber of a gradient mixer and a paclitaxel solution in a high concentration is poured in the second, posterior chamber. The outlet of the gradient mixer is connected to a spray gun. The solution out of the gradient mixer is then sprayed on the balloon catheter with the base coat in a way that increasing paclitaxel concentration is applied. A total of 3.0 $\mu g/mm^2$ paclitaxel is applied. Then the balloon is

dried under slow rotation at room temperature. Finally, the shellac solution is applied as a separate topcoat by a pipetting device on the active agent layer. 1 μg/mm$^2$ shellac is applied. Subsequently, the catheter balloon is thoroughly dried for 30 minutes at 50 °C. The presence of a stent or drug-eluting stent crimped on the balloon does not interfere with the coating process.

Example 1:

Two concentration gradients coating of a catheter balloon with paclitaxel and shellac by a spraying method using a gradient mixer Firstly, 120 mg paclitaxel are solved in 800 μL ethanol and 5 g shellac are solved in 100 mL ethanol stirring for 24 h at room temperature. After this 100 μL of the solution of paclitaxel is mixed with 900 μL of the shellac solution.

The pure shellac solution is applied to the surface of a fold balloon which is rotatably mounted by a pipetting device. Then the fold balloon is dried under slow rotation at room temperature. The base coat contained 1 μg/mm$^2$ shellac on the balloon surface.

The solution containing paclitaxel and shellac is poured in the first chamber of a gradient mixer and the pure paclitaxel solution is poured in the second, posterior chamber. The outlet of the gradient mixer is connected to a spray gun. The solution out of the gradient mixer is then sprayed on the balloon catheter with the base coat in a way that increasing paclitaxel concentration is applied. A total of 3.0 μg/mm$^2$ paclitaxel is applied. During the coating procedure the concentration of said active agent is controlled as follows. When the spray gun is positioned in the middle of balloon, a solution of said active agent with the highest concentration is supplied by gradient mixer. During the spray gun moves from the middle of balloon toward the proximal or distal end of balloon, the concentration of said active agent is reduced slowly by a gradient mixer. When the spray gun is located at the proximal and distal end of balloon, a solution of said active agent with a lowest concentration is supplied by a gradient mixer. The above described concentration controlling is repeated during the spraying procedure. Then the balloon is dried under slow rotation at room temperature. Finally, the shellac solution is applied as a separate topcoat by a pipetting device on the active agent layer. 1 μg/mm$^2$ shellac is applied. Subsequently, the catheter balloon is thoroughly dried for 30 minutes at 50 °C. The presence of a stent or drug-eluting stent crimped on the balloon does not interfere with the coating process.

Example 2:

Two concentration gradients coating of a catheter balloon with paclitaxel and shellac by a pipetting method using a gradient mixer Firstly, 120 mg paclitaxel are solved in 800 μL ethanol and 5 g shellac are solved in 100 mL ethanol stirring for 24 h at room temperature. After this 100 μL of the solution of paclitaxel is mixed with 900 μL of the shellac solution.

The pure shellac solution is applied to the surface of a fold balloon which is rotatably mounted by a pipetting device. Then the fold balloon is dried under slow rotation at room temperature.

The solution containing paclitaxel and shellac is poured in the first chamber of a gradient mixer and the pure paclitaxel solution is poured in the second, posterior chamber. The outlet of the gradient mixer is connected to a pipetting device. The solution out of the gradient mixer is then pipetted on the plain balloon catheter in a way that increasing paclitaxel concentration is applied. A total of 3.0 μg/mm$^2$ paclitaxel is applied at the middle of the balloon where the highest concentration of paclitaxel should be located. During the coating procedure the concentration of paclitaxel is controlled as follows. When the pipetting device is positioned in the middle of balloon, a solution of paclitaxel with the highest concentration is supplied by gradient mixer and a solution of paclitaxel is pipetted 10 times. During the pipetting device moves from the middle of balloon toward the proximal or distal end of balloon, the concentration of paclitaxel is reduced slowly by a gradient mixer and the number of pipetting time is reduced at the same time. When the pipetting device is located at the proximal and distal end of balloon, a solution of paclitaxelwith a lowest concentration is supplied by a gradient mixer and a solution of paclitaxel is pipetted only one time. The above described concentration controlling and pipetting time controlling are repeated during the pipetting procedure. Then the balloon is dried under slow rotation at room temperature. Finally, the shellac solution is applied as a separate topcoat by a pipetting device on the active agent layer. 1 μg/mm$^2$ shellac is applied. Subsequently, the catheter balloon is thoroughly dried for 30 minutes at 50 °C. The presence of a stent or drug-eluting stent crimped on the balloon does not interfere with the coating process.

Reference-example 8:

Coating of a catheter balloon with paclitaxel and shellac Firstly, 120 mg paclitaxel are solved in 800 μL ethyl acetate and 5 g shellac are solved in 100 mL DMSO stirring for 20 h at room temperature.

The pure shellac solution is applied to the surface of a fold balloon which is rotatably mounted by a pipetting device. Then the balloon is dried under slow rotation at room temperature. Said layer contained 2 μg/mm$^2$ shellac on

the balloon surface.

The paclitaxel solution is applied on the existing layer of shellac. Thereby the paclitaxel solution soaked into the shellac layer and a gradient develops where the highest concentration of shellac is on top of the coating. A total of 3.0 μg/mm$^2$ paclitaxel is applied. Then the balloon is dried under slow rotation at 50 °C.

Reference-example 9: Biological test 1

Eight polish domestic pigs of 35-42 kg body weight were included in the study in which 24 paclitaxel eluting balloons were deployed. Procedures were carried out in the Animal Cath Lab of American Heart of Poland between November 3$^{th}$ and 14$^{th}$ of 2010. The appropriate approval of regional Bioethical Committee was obtained. The three coronary arteries (LAD, LCX, RCA) of each animal were randomly assigned in 1:1:1 ratio to either study group.

Three studied catheters with the following coatings were evaluated:

1. 3.0 μg/mm$^2$ Paclitaxel + 0.3 μg/mm$^2$ Alpha Linolen + 0.3 μg/mm$^2$ Boswellic acid
2. 3.0 μg/mm$^2$ Paclitaxel + 0.3 μg/mm$^2$ z Alpha Linolen
3. 3.0 μg/mm$^2$ Paclitaxel and 3.0 μg/mm$^2$ shellac coated according to reference-example 1

All studied balloons were 3.0 mm in diameter and 20 mm length.

Methods

Animals received antiplatelet therapy consisting of acetylsalicylic acid and clopidogrel three days prior to intervention and throughout the study. Under general anesthesia femoral artery access through 6 F sheath was gained for stent introduction and implantation into the two different coronary arteries. All balloons were implanted under "live" quantitative angiography analysis guidance at an inflation pressure which ensured a balloon/artery diameter ratio of 1.15 : 1.0.

The Quantitive Coronary Angiography (QCA) analysis was performed with the use of CMS-QCA software (Medis) and angiograms were recorded in DICOM format. Two contralateral projections were chosen for stent assessment. At pre-determined time points the animals were euthanized. The hearts were harvested as quickly as possible after euthanasia, using precautions to avoid damage to the study vessels. The hearts were examined for abnormal findings and were labeled with the animal identification number, protocol number and date of collection. The hearts were flushed with normal saline until cleared of blood and then pressure-perfusion fixed at 80-100 mmHg with 10% neutral buffered formalin (NBF). Samples of abnormal tissues were collected and undergo immersion fixation with 10% NBF. All study vessel segment were labeled with the animal identification number, protocol number, tissue types and date of collection. All tissues were placed in containers and frozen in dry ice in -68 C and sent to the HPLC test site. The heart for each animal was placed in its own separate container.

HPLC Analysis

The paclitaxel concentration of plasma, LAD, LCx and RCA were measured by highperformance liquid chromatography (AnaKat Institut für Biotechnologie GmbH, Berlin, Germany, analysis blinded to sample origin). Briefly, after thawing, the tissues were weighed at ambient temperature and, depending on the weights, different volumes of ethanol were added to the samples (sufficient ethanol to cover the tissue completely). The samples were then treated with ultrasound for 40 min. About 200 ml samples were centrifuged.

A calibration line was produced in the range between 50 and 5000 ng/ml. The samples for the calibration line were prepared by dilution of a stock solution with a concentration of 1000 mg/ml. Aliquots of all samples (samples from tissue and calibration line) were transferred into autosampler vials and the same volume of 0.1 % formic acid was added. The flow rate of the highperformance liquid chromatography system was 0.2 ml/min through a column of ODs Hypersil (ThermoElectron Corporation, Thermo Scientific, Waltham, Massachusetts, USA), particle size 5 m, pore size 120A°. The isocratic mobile phase consisted of 70% methanol containing formic acid (0.1%). Paditaxel was detected by mass spectrometry in multiple reaction-monitoring mode with a transition of paclitaxel from 854 to 105AMU. The tissue paclitaxel concentration was expressed in μg/g.

Pre-Operative Procedures

After an overnight fast, the animals were pre anesthetized with a mixture based on body weight. These drugs include: Atropine (1 mg / 20 kg sc.), Ketamine (4 mL / 10 kg im.) and Xylazine (1 ml / 10 kg im). The injection was given intramuscularly in either the neck or rear muscle quadrant by a qualified animal technologist. The animal was transferred

to the preparation room, where an intravenous line was placed in the auricular marginal vein, and intravenous fluids (lactated ringers or 0.9% saline) were administered throughout the procedure. Anti-arrhythmics were added to these IV fluids (Lidocaine 200mg/liter, Metoprolol 5mglliter). When the animal reached an adequate anesthetic plane (gas mask with 1-3% isoflurane), it was intubated with an appropriate size endotracheal tube, which was tied into place and the cuff inflated to prevent leakage. The animal was then transferred to the cath lab, placed on the table and attached to the anesthesia and ventilator unit.

Procedures

In total 24 balloons were deployed, eight of group 1 and 2 and 8 of group 3 (according to the invention). Each of them was inspected before delivery. No signs of structure abnormality were noticed. Balloons were easily introduced into the selected arterial segment through femoral artery access and successfully deployed in the desired segment after live QCA guidance to ensure balloon/artery ratio 1,1 : 1. All tested balloons were inflated for 60s. Due to overstretch in 3 case dissections post balloon inflation were visible, although the vessel remained opened and distal flow was not impaired, therefore a stent implantation was not necessary.

Follow - up

The animals were scheduled for 1 hour, 1, 3 and 7 days (2 pigs per time period). Within the entire follow-up period, neither death nor major adverse events cardiac events were noted. All animals remained in good general condition and a steady bodyweight increase was observed.

Statistical analysis

Results are expressed as mean and standard deviation (SD). Normal distribution of variables was verified by Kolmogorov-Smirnov test. The variance uniformity was verified with the use of Levene test. Angiographic and HPLC anaylsis data were analyzed using ANOVA tests. In case of skewed distribution or non-uniformity of variance a nonparametric Kruskal-Wallis and U Mann-Whitney tests were used. The p-value <0.05 was considered statistically significant.

Results

Baseline vessel and balloon deployment characteristics: There were no differences in the baseline QCA results such as vessels baseline, reference diameter, minimal lumen diameter, balloon diameter and stent to artery ratio in the whole group as well as within each time period between the studied groups (Table 1) The average overstretch was 210 % and was reproducible among groups. All tested balloons stayed in circulation for 3 minutes ± 30 seconds.

*Table 1.* Baseline QCA vessel characteristics

| mm All | RD [mm Average ± SD | MLD Average ± SD | Balloon Average ± SD | S-2-A Average ± SD |
|---|---|---|---|---|
| | | Total n=24 | | |
| Group 1 | 2,68 ± 0,3 | 2,46 ± 0,3 | 2,97 ± 0,1 | 1,11 ± 0,1 |
| Group 2 | 2,75 ± 0,2 | 2,55 ± 0,5 | 2,93 ± 0,1 | 1,07 |
| Group 3 | 2,67 ± 0,2 | 2,43 ± 0,4 | 3,03 ± 0,2 | 1,14 |
| P ANOVA | ns | ns | ns | ns |
| | | 1 hour (0) n=6 | | |
| Group 1 | 2,68 ± 0,3 | 2,46 ± 0,3 | 2,97 ± 0,1 | 1,11 ± 0,1 |
| Group 2 | 2,75 ± 0,2 | 2,55 ± 0,5 | 2,93 ± 0,1 | 1,07 |
| Group 3 | 2,67 ± 0,2 | 2,43 ± 0,4 | 3,03 ± 0,2 | 1,1 |
| P ANOVA | ns | ns | ns | ns |
| | | 1 day n=6 | | |
| Group 1 | 2,21 ± 0,2 | 2,11 ± 0,2 | 2,65 ± 0,1 | 1,21 ± 0,1 |
| Group 3 | 2,48 ± 0,2 | 2,22 ± 0,6 | 2,77 ± 0,1 | 1,12 ± 0,1 |
| Group 2 | 2,50 ± 0,1 | 2,45 ± 0,1 | 2,81 | 1,13 |
| P ANOVA | ns | ns | ns | ns |
| | | 3 days n= 6 | | |
| Group 1 | 2,67 ± 0,1 | 2,43 ± 0,2 | 2,99 ± 0,2 | 1,12 |
| Group 3 | 2,59 ± 0,2 | 2,35 ± 0,2 | 2,90 | 1,12 ± 0,1 |
| Group 2 | 2,56 ± 0,1 | 2,19 | 2,86 ± 0,1 | 1,12 ± 0,1 |
| P ANOVA | ns | ns | ns | ns |
| | | 7 days n=6 | | |
| Group 3 | 2,58 ± 0,2 | 2,37 ± 0,5 | 2,89 ± 0,1 | 1,13 ± 0,1 |
| Group 1 | 2,68 ± 0,3 | 2,49 ± 0,1 | 2,86 ± 0,1 | 1,07 ± 0,1 |
| Group 2 | 3,00 | 2,83 ± 0,1 | 3,00 ± 0,1 | 1,00 |

Paclitaxel concentration analysis There was a significantly higher intramural vessel concentration of paclitaxel in group 3 at 1 hour observation. At 1 day, although not statistically significant it remained numerically much higher. At 3 and 7 days, in the group 3, the concentration decreased to 1 μg/g and to a not recognizable level in groups 1 and 2 (Figure 1, Table 2). These results were sustained in percentage of initial loading dose analysis (Figure 2).

*Table 2*. Vessel intramural paclitaxel Concentration

| μg/g | Group 3 N=2 | Group 1 N=2 | Group 2 N=2 | ANOVA p |
|---|---|---|---|---|
| **1 hour** | 43.8 ± 14.9 | 0.2 ± 0.2 | 1.3 ±1.9 | 0.02 |
| **1 day** | 107.6 ± 97 | 1.1 | 0 | 0.2 |
| **3days** | 4.1 ± 6 | 1.8 ± 2.5 | 1.2 ± 0.3 | 0.73 |
| **7 days** | 4.7 ± 6.6 | 0 | 0 | 0.4 |

All tested balloons were easily introduced and deployed at study sites. No delivery or withdrawal problems occurred. The balloon diameters at nominal inflation reached their designed diameter. Adverse events were noted neither after procedures nor at follow up. On autopsy no macroscopic signs of myocardial infarction, or inflammation within studied site were observed. The studied baseline vessel characteristics were similar between groups with regard to reference diameter and minimal lumen diameter. Most importantly the stent to artery ratio of 1.1:1 resulted in similar overstretch between the studied groups. All inflation was performed for 60 s and all balloon remained within the same period of time in circulation. Basing on previous studies this overstretch and inflation time should definitely provide proper and reproducible conditions for paclitaxel delivery (1,2).

In the HPLC analysis there was a significantly higher paclitaxel tissue concentration at 1 hour observation in the group receiving the balloon according to the invention when compared to group 1 and 2. At 24 hour this difference although not significant, (caused mainly by high standard deviation), numerically remained very different. At 3 and 7 days, in the paclitaxel eluting, shellac covered balloons the concentration decreased. Said paclitaxel tissue concentration and loading doses percentages are higher to dose found in the Dior paclitaxel eluting balloons and similar to Sequent Please assessed in previous studies (2, 3). On the other hand the paclitaxel delivery of the balloons of group 1 and 2 was not sufficient. The concentrations were very low at all study points, to a level not distinguishable by HPLC at 3 and 7 days. The tissue paclitaxel concentration levels were even lower to this observed in paclitaxel balloons without any carrier molecule (1).

Reference-example 10: Biological test 2

14 juvenile healthy farm pigs underwent catheter based paclitaxel eluting balloon deployment of the coronary arteries (please refer to the implant matrix of Table 3a and 3b below for the assignment of catheter balloons to the coronary arteries of each individual animal). In summary, 5 plain, uncoated catheter balloons (POBA), 10 DIORII and 10 PRIMUS were deployed in 25 coronary arteries. Pigs were euthanized 7 and 28 days later (n=5 per time point; POBA 28 days only) and subjected to gross pathology examination of major organs, especially intra-thoracal organs and pleura. Hearts were explanted and myocardial tissue as well as the treated and adjacent vessel segments were excised and histopathologically examined.

The following myocardial tissues /arteries were analysed by histopathology:

1. Treated coronary arteries within the region of balloon deployment
2. Proximal and distal located vessel segments adjacent to treated segments
3. Randomly allocated and grossly suspect myocardial tissues

The three coronary arteries (LAD, LCX, RCA) of each animal were randomly assigned to either study group (Table 3).

Three studied catheters with the following coatings were evaluated:

1. Primus - Paclitaxel Coated Coronary PTCA Catheter, coated with 3.0 $\mu g/mm^2$ Paclitaxel and 3.0 $\mu g/mm^2$ shellac according to reference-example 8.
2. Dior II - Paclitaxel Coated Angioplasty Balloon, Eurocor.
3. POBA - Plain old balloon angioplasty, which served as the control (no coating on the balloon).

All studied balloons were 3.0 mm in diameter and 20 mm length.

*Table 3*. 7-day and 28-day group implant matrix

| Group | CV Number | Animal Number | Vessel/Treatment | | |
|---|---|---|---|---|---|
| | | | LAD | LCX | RCA |
| 7-day group | 28191 | 3906 | Dior II | | |
| | 28192 | 3908 | Primus | Dior II | |
| | 28193 | 3919 | | | Primus |
| | 28194 | 3938 | Dior II | | |
| | 28195 | 3947 | | | Dior II |
| | 28196 | 3959 | | | Primus |
| | 28197 | 3970 | | Primus | Dior II |

(continued)

| Group | CV Number | Animal Number | Vessel/Treatment | | |
|---|---|---|---|---|---|
| | | | LAD | LCX | RCA |
| 28-day group | 28426 | 3907 | Primus | Dior II | POBA |
| | 28427 | 3914 | POBA | | Primus |
| | 28428 | 3931 | Dior II | | POBA |
| | 28429 | 3934 | POBA | | Primus |
| | 28430 | 3953 | | Primus | Dior II |
| | 28431 | 3968 | | Primus | Dior II |
| | 28432 | 3972 | Dior II | POBA | |
| Abbreviations: LAD = left anterior descending artery, LCX = left circumflex artery , RCA = right coronary artery | | | | | |

Methods

The coronary vessels with branches intact were dissected and carefully removed from each heart. Using angiography videos, still frame images were created and balloon-treated regions were labeled. Using the branches as anatomic markers and the fact that each region of treatment was known to be 2 cm long, the location of treatment was estimated and sectioned to include 0.5 cm proximal and 0.5 cm distal to the treatment site.

Hearts were bread-loafed at 1 cm intervals parallel to the atrio-ventricular groove. Full thickness myocardial sections were then sampled circumferentially in the distribution of the major coronary arteries to include the anterior, lateral, posterior, and septal wall at two levels (mid and apical). In addition, the right ventricle was also sampled at the mid and apical levels. Any grossly suspect area of myocardium were also sampled and processed for evaluation. Sections from all non-target organs, including samples from the left and right lungs, liver, left and right kidneys, spleen, mediastinal lymph nodes, and rib (for bone marrow) were also taken. All sections were examined for the presence of infarct and thromboembolus.

Paraffin Embedding

For paraffin histology, the tissue was processed through a graded series of alcohols and xylenes. After dehydration, vessels were cut into a proximal non-treated, mid treated, and distal non-treated region. The treated region was further cut into 2-5 segments at 3-4 mm intervals and sequentially placed in a paraffin block with the most proximal portion offset for orientation (referenced as section A). Histologic sections were cut at 4 to 6 $\mu$m, mounted on charged slides, and stained with hematoxylin and eosin (H&E) and an elastin (Movat Pentachrome) stain. All sections were examined by light microscopy for the presence of inflammation, thrombus, neointimal formation and vessel wall injury. All myocardial and non-target organ sections were placed in separate cassettes and embedded in paraffin, sectioned and stained with H&E only.

Morphometry

Morphometry measurements were performed on the 2-3 histological sections (except where only 1 was available) with the greatest medial injury. In cases where the 'distal' sections showed more injury than the 'treated' sections, the 'distal' sections were used to represent the 'treated' sections. The morphometry software (IP Lab for Mac OS X, Scanalytics, Rockville, MD) was calibrated at 2x objective using 2.0 mm linear and circular NIST traceable micrometers. Klarmann Rulings, Inc., (Manchester, New Hampshire) certified the micrometer graduations.

The vessel cross-sectional areas (external elastic lamina [EEL] and internal elastic lamina [IEL], and lumen) were measured. Minimum and maximum neointimal thickness and minimum and maximum medial thickness (as determined visually) were also measured. Mean neointimal thickness/thrombus burden and mean medial thickness were calculated, as well as % Stenosis, medial area, and neointimal area with the following formulas:

$$\% \text{ Stenosis} = [1 - (\text{Lumen Area}/\text{IEL Area})] * 100$$

$$\text{Neointimal area} = \text{IEL area} - \text{Lumen area}$$

$$\text{Medial area} = \text{EEL area} - \text{IEL area}$$

Histologic Evaluation

The same sections selected for morphometry were evaluated for injury using the semi-quantitative scoring criteria listed in Table 4. Each section on the slide is referenced alphabetically beginning at the proximal end (offset section) with the letter A.

To assess arterial injury and healing, ordinal data was collected for multiple parameters to include endothelialization, platelets/fibrin, inflammation, red blood cells, injury, proteoglycans/collagens, medial smooth muscle cell loss (depth and circumference), and adventitial fibrosis. These parameters were semi-quantified using a scoring system of 0 to 4: 0 = none; 1= minimal, 2 = mild; 3 = moderate; and 4 = severe (or marked).

*Table 4.* Semi-quantitative Analysis Evaluation of Pathologic Changes in Vessels

| Scored Parameter | 0 none) | 1 (minimal) | 2 (mild) | 3 (moderate) | 4 (marked) |
|---|---|---|---|---|---|
| TISSUE MATRIX | | | | | |
| Fibrin (Intima/Media) | none | Minimal, focal | Mild, multifocal | Moderate, regionally diffuse | Severe, marked diffuse |
| MEDIAL SMOOTH MUSCLE CELL LOSS | | | | | |
| Medial SMC loss (Depth) | none | smooth muscle loss <25 % of medial thickness | 25-50% of medial thickness | 51-75% of medial thickness | >75% of medal thickness |
| Medial SMC loss (Circumference) | none | <25% of the area | 25-50% | 51-75% | >75% |
| Abbreviations: SMC= smooth muscle cell | | | | | |

Statistical analysis

The data are reported as the means$\pm$standard deviation. Statistical differences between morphometry (parametric) values are reported using a one-way analysis of variance (ANOVA) (JMP software, Cary, NC). Ordinal data (non-parametric histology scores) are compared using the Wilcoxon/Kruskal-Wallis (Rank Sums) test. P values $\leq$0.05 were considered significant (bolded on tables). If statistical significance was reached, a further sub group analysis (each pair, Student's T-test for continuous variables, or Wilcoxon/Kruskal-Wallis for ordinal data) was performed comparing test groups means to control group means. In comparison of ordinal data, the Wilcoxon/Kruskal-Wallis is used for analysis in conjunction with the Bonferroni correction to determine a more stringent p-value for statistical significance ($p \leq \frac{0.05}{\#GroupComparisons} = 0.0083$ or less).

Results

*Morphometric and histological findings*

The morphometric data representing vessel size and dimensions (EEL, IEL area) show that vessel size was similar among groups, with slightly larger numerical values in the PRIMUS groups compared to the DIORII at 28 days. Percent stenosis (lumen obstruction) was greater in the DIORII at 7 days. Both groups showed an increase in this parameter at 28 compared to 7 days follow-up (Table 5).

*Table:*. Stenosis (%)

| Treatment Group | Treated Regions | | Non-Treated Regions | |
|---|---|---|---|---|
| | | | Proximal | Distal |
| | 7 days | 28 days | 28 days | 28 days |
| PRIMUS | 0.94±1.13<br>- | 7.52±6.54<br>-- | 1.73±2.11<br>- | 3.84±3.04<br>- |
| DIOR II | 4.13±3.91<br>-- | 7.41±8.15<br>-- | 4.88±7.32<br>-- | 8.52±14.40<br>--- |
| POBA | | 1.43±1.99<br>-- | 0.19±0.43<br>-- | 1.14±1.05<br>-- |
| Abbreviations: 0= none, - = 0.01 - 4.00 %, - - = 4.01 - 8.00 %, - - - = 8.01 - 12.00 % | | | | |

The loss of endothelial cells was similar among groups. Fibrin deposition was greatest in the DIORII at 7 days but was absent at 28 days. On the contrary, PRIMUS showed elevated fibrin deposition at 28 days (Table 6). Similar trends were observed for medial smooth muscle cell loss between groups and time points. Fibrin score and medial SMC loss as indirect measures of device efficacy were elevated in the PRIMUS at 28 days compared to DIOR II. PRIMUS provides sustained paclitaxel action to prevent proliferation of smooth muscle cells (Table 7).

*Table 6:* Healing Parameter1 - Fibrin Score

| Treatment Group | Treated Regions | | Non-Treated Regions | |
|---|---|---|---|---|
| | | | Proximal | Distal |
| | 7 days | 28 days | 28 days | 28 days |
| PRIMUS | 0.10±0.22<br>+ | 0.22±0.45<br>+ | 0.00±0.00<br>0 | 0.00±0.00<br>0 |
| DIOR II | 0.44±0.65<br>+ | 0.00±0.00<br>0 | 0.00±0.00<br>0 | 0.00±0.00<br>0 |
| POBA | | 0.00±0.00<br>0 | 0.00±0.00<br>0 | 0.00±0.00<br>0 |
| Abbreviations: 0= none, + = 0.01 - 0.50 score, ++ = 0.51 - 1.00 score, +++ = 1.01 - 1.50 score, ++++ = 1.51 - 2.0 score. | | | | |

*Table 7.*: Healing Parameter 2 - Medial SMC Loss Score (28 days)

| Treatment Group | Treated Regions | | Non-Treated Regions | | | |
|---|---|---|---|---|---|---|
| | | | Proximal | | Distal | |
| | Depth | Circumference | Depth | Circumference | Depth | Circumference |
| PRIMUS | 1.70±1.89<br>++++ | 0.87±0.89<br>++ | 0.50±0.87<br>+ | 0.20±0.27<br>+ | 0.90±1.24<br>++ | 0.70±0.84<br>++ |
| DIOR II | 0.70±0.76<br>++ | 0.50±0.35<br>+ | 0.00±0.00<br>0 | 0.00±0.00<br>0 | 0.90±1.24<br>++ | 0.40±0.65<br>+ |
| POBA | 1.20±1.44<br>+++ | 0.50±0.50<br>+ | 0.20±0.45<br>+ | 0.10±0.22<br>+ | 0.10±0.22<br>+ | 0.10±0.22<br>+ |
| Abbreviations: 0= none, + = 0.01 - 0.50 score, ++ = 0.51 - 1.00 score, +++ = 1.01 - 1.50 score, ++++ = 1.51 - 2.0 score. | | | | | | |

There was no indication of myocardial infarction in any of the treated hearts. However, there were some observations demonstrating DCB-associated reactions of the myocardium and myocardial vessels distal to the treated

vessels. Importantly, in the acute/short term phase (7 days after treatment) PRIMUS showed less myocardial and vascular changes compared to DIORII (Table 8).

*Table 8*. Myocardial finding (7 days)

| Treatment Group | PRIMUS | DIOR II |
|---|---|---|
| % observations tissue scarring/ischemia | 3.6-(5/112) | 4.5-(4/112) |
| % observations distal emboli/vascular changes | 3.6-(4/112) | 6.3 -- (7/112) |
| Abbreviations: 0= none, - = 0.01 - 4.00 %, - - = 4.01 - 8.00 %, - - - = 8.01 - 12.00 % | | |

*Hypothetical assessment of treatment effect and safety*

Considering the healing parameters as more sensitive to address device efficacy, this study shows that prolonged paclitaxel effect is more evident in PRIMUS than in DIORII. On the other hand, DIORII showed augmented paclitaxel effect in the early phase of the study, indicating that effective paclitaxel uptake is provided but is of limited duration. This may be attributed to the different coating technologies used on these DCBs. DIORII uses shelloic acid as coating matrix which primarily acts as a barrier during systemic delivery. At expansion small crystal fragments of paclitaxel and shelloic acid are transmitted into the vessel wall. However, after balloon deflation and retraction micro-particles may be carried away by the blood circulation and thus lower the efficacy of this technology. PRIMUS uses a novel gradient coating method. The shellolic acid is not evenly mixed with the drug. The drug is coated on top and is only slightly embedded into the surface of the shellolic base coating. As a result, much less crystal fragments of shellolic acid are transferred into the arterial tissue, rather condensed paclitaxel release from the surface of its carrier matrix may facilitate intra-cellular drug uptake and retention and may therefore explain the delayed vascular healing response as noted by the 28-day data.

Both DCBs exhibited focal scar formation of the downstream myocardium within the expected ranges. In this regard, the 7-day data may be more relevant, as it reflects the short-term myocardial response after DCB deployment and may better relate to coating induced micro-emboli and in consequence of special clinical interest. At this early time point, DIORII showed a greater frequency of acute myocardial changes as compared to PRIMUS.

Conclusions

The active agent eluting balloon according to the invention showed promising results with regard to drug deliverability to the vessel wall, with even higher levels of tissue paclitaxel concentration when compared with previously published data on clinically approved active agent coated balloons.

At 28 days following DCB deployment PRIMUS showed greater efficacy in drug-delivery to the vessel wall compared to DIORII. On the contrary, DIORII seemed effective for up to 7 days with loss of efficacy at 28 days. All devices exhibited a safety profile within the expected range. Further assessment of the DCBs at long-term follow-up is needed to confirm these findings.

References:

1. Gray WA, Granada JF. Drug-coated balloons for the prevention of vascular restenosis. Circulation; 121:2672-80.
2. Posa A et al. Attainment of local drug delivery with paclitaxel-eluting balloon in porcine coronary arteries. Coron Artery Dis 2008; 19:243-7.
3. Scheller B, Speck U, Schmitt A, Bohm M, Nickenig G. Addition of paclitaxel to contrast media prevents restenosis after coronary stent implantation. J Am Coll Cardiol 2003;42: 141 5-20.

**Claims**

1. A catheter balloon comprising a coating with an active agent and shellac, wherein the coating comprises a vertical concentration gradient of the active agent and a horizontal gradient of the active agent and wherein for the vertical concentration gradient the concentration of the active agent increases from the surface of the balloon to the top or the surface of the coating.

2. Catheter balloon according to claim 1, wherein the concentration gradient of the active agent is in the layer of shellac as a matrix substance.

3. Catheter balloon according to claim 1 or 2, wherein the coating comprises further a base coat of shellac as a first layer under the concentration gradient of the active agent.

4. Catheter balloon according to claim anyone of claim 1 - 3, wherein the coating comprises further a top coat of shellac

5. Catheter balloon according to anyone of claim 1 - 4, wherein the active agent is an antiproliferative, immunosuppressive, anti-angiogenic, anti-inflammatory, and/or anti-thrombotic agent.

6. Catheter balloon according to anyone of claim 1 - 5, wherein the active agent is selected from the group consisting of:

abciximab, acemetacin, acetylvismione B, aclarubicin, ademetionine, adriamycin, aescin, afromosone, akagerine, aldesleukin, amidorone, aminoglutethimide, amsacrine, anakinra, anastrozole, anemonin, anopterine, antimycotics antithrombotics, apocymarin, argatroban, aristolactam-All, aristolochic acid, ascomycin, asparaginase, aspirin, atorvastatin, auranofin, azathioprine, azithromycin, baccatin, bafilomycin, basiliximab, bendamustine, benzocaine, berberine, betulin, betulinic acid, bilobol, bisparthenolidine, bleomycin, combrestatin, Boswellic acids, bruceanol A, B and C, bryophyllin A, busulfan, antithrombin, bivalirudin, cadherins, camptothecin, capecitabine, o-carbamoyl-phenoxyacetic acid, carboplatin, carmustine, celecoxib, cepharanthin, cerivastatin, CETP inhibitors, chlorambucil, chloroquine phosphate, cicutoxin, ciprofloxacin, cisplatin, cladribine, clarithromycin, colchicine, concanamycin, coumadin, C-type natriuretic peptide (CNP), cudraisoflavone A, curcumin, cyclophosphamide, ciclosporin A, cytarabine, dacarbazine, daclizumab, dactinomycin, dapsone, daunorubicin, diclofenac, 1,11-dimethoxycanthin-6-one, docetaxel, doxorubicin, daunamycin, epirubicin, erythromycin, estramustine, etoposide, everolimus, filgrastim, fluroblastin, fluvastatin, fludarabine, fludarabine-5'-dihydrogen phosphate, fluorouracil, folimycin, fosfestrol, gemcitabine, ghalakinoside, ginkgol, ginkgolic acid, glycoside 1a, 4-hydroxyoxycyclo phosphamide, idarubicin, ifosfamide, josamycin, lapachol, lomustine, lovastatin, melphalan, midecamycin, mitoxantrone, nimustine, pitavastatin, pravastatin, procarbazine, mitomycin, methotrexate, mercaptopurine, thioguanine, oxaliplatin, irinotecan, topotecan, hydroxycarbamide, miltefosine, pentostatin, pegaspargase, exemestane, letrozole, formestane, mycophenolate mofetil, β-lapachone, podophyllotoxin, podophyllic acid-2-ethyl hydrazide, molgramostim (rhuGM-CSF), peginterferon α-2b, lenograstim (r-HuG-CSF), macrogol, selectin (cytokine antagonist), cytokinin inhibitors, COX-2 inhibitor, angiopeptin, monoclonal antibodies inhibiting muscle cell proliferation, bFGF antagonists, probucol, prostaglandins, 1-hydroxy-11-methoxycanthin-6-one, scopoletin, NO donors, pentaerythrityl tetranitrate and sydnoimines, tamoxifen, staurosporine, β-estradiol, α-estradiol, estriol, estrone, ethinyl estradiol, medroxyprogesterone, estradiol cypionates, estradiol benzoates, tranilast, kamebakaurin and other terpenoids used in cancer therapy, verapamil, tyrosine kinase inhibitors (tyrphostins), paclitaxel, 6-α-hydroxy-paclitaxel, taxoteres, mofebutazone, lonazolac, lidocaine, ketoprofen, mefenamic acid, piroxicam, meloxicam, penicillamine, hydroxychloroquine, sodium aurothiomalate, oxaceprol, β-sitosterol, myrtecaine, polidocanol, nonivamide, levomenthol, ellipticine, D-24851 (Calbiochem), colcemid, cytochalasin A-E, indanocine, nocodazole, bacitracin, vitronectin receptor antagonists, azelastine, guanidyl cyclase stimulator, tissue inhibitor of metal proteinase-1 and -2, free nucleic acids, nucleic acids incorporated into virus transmitters, DNA and RNA fragments, plasminogen activator inhibitor 1, plasminogen activator inhibitor 2, antisense oligonucleotides, VEGF inhibitors, IGF-1, active agents from the group of antibiotics, cefadroxil, cefazolin, cefaclor, cefoxitin, tobramycin, gentamicin, penicillins, dicloxacillin, oxacillin, sulfonamides, metronidazole, enoxaparin, heparin, hirudin, PPACK, protamine, prourokinase, streptokinase, warfarin, urokinase, vasodilators, dipyramidole, trapidil, nitroprussides, PDGF antagonists, triazolopyrimidine, seramin, ACE inhibitors, captopril, cilazapril, lisinopril, enalapril, losartan, thioprotease inhibitors, prostacyclin, vapiprost, interferon α, β and γ, histamine antagonists, serotonin blockers, apoptosis inhibitors, apoptosis regulators, halofuginone, nifedipine, tocopherol, tranilast, molsidomine, tea polyphenols, epicatechin gallate, epigallocatechin gallate, leflunomide, etanercept, sulfasalazine, tetracycline, triamcinolone, mutamycin, procainimide, retinoic acid, quinidine, disopyrimide, flecainide, propafenone, sotalol, natural and synthetically obtained steroids such as bryophyllin A, inotodiol, maquiroside A, ghalakinoside, mansonine, strebloside, hydrocortisone, betamethasone, dexamethasone, non-steroidal substances (NSAIDS), fenoprofen, ibuprofen, indomethacin, naproxen, phenylbutazone, antiviral agents, acyclovir, ganciclovir zidovudine, clotrimazole, flucytosine, griseofulvin, ketoconazole, miconazole, nystatin, terbinafine, antiprotozoal agents, chloroquine, mefloquine, quinine, natural terpenoids, hippocaesculin, barringtogenol-C21-angelate, 14-dehydroagrostistachin, agroskerin, agrostistachin, 17-hydroxyagrostistachin, ovatodiolids, 4,7-oxycycloanisomelic acid baccharinoids B1, B2, B3 and B7, tubeimoside, bruceantinoside C, yadanziosides N and P, isodeoxyelephantopin, tomenphantopin A

and B, coronarin A,B C and D, ursolic acid, hyptatic acid A, iso-iridogermanal, maytenfoliol, effusantin A, excisanin A and B, longikaurin B, sculponeatin C, kamebaunin, leukamenin A and B, 13,18-dehydro-6-alpha-senecioyloxychaparrin, taxamairin A and B, regenilol, triptolide, cymarin, hydroxyanopterine, protoanemonin, cheliburin chloride, sinococuline A and B, dihydronitidine, nitidine chloride, 12-β-hydroxypregnadien-3,20-dione, helenalin, indicine, indicine-N-oxide, lasiocarpine, inotodiol, podophyllotoxin, justicidin A and B, larreatin, malloterin, mallotochromanol, isobutyrylmallotochromanol, marchantin A, maytansin, lycoridicin, margetine, pancratistatin, liriodenine, oxoushinsunine, periplocoside A, deoxypsorospermin, psychorubin, ricin A, sanguinarine, manwu wheat acid, methylsorbifolin, chromones of spathelia, stizophyllin, dihydrousambaraensine, hydroxyusambarine, strychnopentamine, strychnophylline, usambarine, usambarensine, liriodenine, daphnoretin, lariciresinol, methoxylariciresinol, syringaresinol, sirolimus (rapamycin), biolimus A9, pimecrolimus, everolimus, zotarolimus, tacrolimus, fasudil, epothilones, somatostatin, roxithromycin, troleandomycin, simvastatin, rosuvastatin, vinblastine, vincristine, vindesine, teniposide, vinorelbine, trofosfamide, treosulfan, temozolomide, thiotepa, tretinoin, spiramycin, umbelliferone, desacetylvismione A, vismione A and B, zeorin.

**7.** Catheter balloon according to claim 6, wherein the active agent is selected from the group consisting of:

paclitaxel, taxanes, docetaxel, rapamycin, biolimus A9, pimecrolimus, everolimus, zotarolimus, tacrolimus, fasudil and epothilones.

**8.** Catheter balloon according to claim 7, wherein the active agent is paclitaxel.

**9.** Method for coating a catheter balloon of claim 1 comprising the following steps:

A) providing an uncoated catheter balloon; and
B) providing a solution of an active agent and providing a solution of shellac; and
C) coating the surface of the catheter balloon with the solution of shellac; and
D) applying the solution of the active agent so that two different concentration gradients in vertical and horizontal direction of the active agent in shellac are induced using a gradient mixer and subsequently
E) drying the coated catheter balloon.

**10.** Method according to claim 9, wherein the solution of the active agent is prepared using a solvent wherein the solubility of the active agent, preferably paclitaxel, is greater than of shellac.

**11.** Method according to claim 9 or 10, wherein the solution of the active agent is prepared in ethyl acetate.

**12.** Method according to any claim of 9 to 11, wherein the active agent is paclitaxel.

**13.** Method according to anyone of claim 9 to 12, wherein the active agent containing solution is applied by means of spray coating, brush coating, vapour deposition or pipetting.

**14.** Dilatation catheter comprising the coated catheter balloon according to anyone of claim 1-7.

## Patentansprüche

**1.** Ein Ballonkatheter umfassend eine Beschichtung mit einem Wirkstoff und Schellack,
wobei die Beschichtung einen vertikalen Konzentrationsgradienten des Wirkstoffs und einen horizontalen Gradienten des Wirkstoffs umfasst und wobei für den vertikalen Konzentrationsgradienten die Konzentration des Wirkstoffs von der Oberfläche des Ballons zur Oberseite oder zur Oberfläche der Beschichtung zunimmt.

**2.** Katheterballon gemäß Anspruch 1, wobei der Konzentrationsgradient des Wirkstoffs in der Schicht des Schellacks als eine Matrixsubstanz ist.

**3.** Katheterballon gemäß Anspruch 1 oder 2, wobei die Beschichtung weiter eine Basisschicht aus Schellack als eine erste Schicht unter dem Konzentrationsgradienten des Wirkstoffs umfasst.

**4.** Katheterballon gemäß einem der Ansprüche 1 - 3, wobei die Beschichtung weiter eine Deckschicht aus Schellack umfasst.

**5.** Katheterballon gemäß einem der Ansprüche 1 - 4, wobei der Wirkstoff ein antirestenotisches, antiproliferatives, immunsuppressives, anti-angiogenes, antiinflammatorisches und/oder anti-thrombotisches Agens ist.

**6.** Katheterballon gemäß einem der Ansprüche 1 - 5, wobei der Wirkstoff ausgewählt ist aus der Gruppe bestehend aus:

Abciximab, Acemetacin, Acetylvismion B, Aclarubicin, Ademetionin, Adriamycin, Aescin, Afromoson, Akagerin, Aldesleukin, Amidoron, Aminoglutethimid, Amsacrin, Anakinra, Anastrozol, Anemonin, Anopterin, Antimykotika Antithrombotika, Apocymarin, Argatroban, Aristolactam-All, Aristolochsäure, Ascomycin, Asparaginase, Aspirin, Atorvastatin, Auranofin, Azathioprin, Azithromycin, Baccatin, Bafilomycin, Basiliximab, Bendamustin, Benzocain, Berberin, Betulin, Betulinsäure, Bilobol, Bisparthenolidin, Bleomycin, Bombrestatin, Boswellinsäuren, Bruceanol A, B und C, Bryophyllin A, Busulfan, Antithrombin, Bivalirudin, Cadherine, Camptothecin, Capecitabin, o-Carbamoylphenoxyessigsäure, Carboplatin, Carmustin, Celecoxib, Cepharanthin, Cerivastatin, CETP-Inhibitoren, Chlorambucil, Chlorochinphosphat, Cicutoxin, Ciprofloxacin, Cisplatin, Cladribin, Clarithromycin, Colchicin, Concanamycin, Coumadin, C-Typ-natriuretisches Peptid (CNP), Cudraisoflavon A, Curcumin, Cyclophosphamid, Ciclosporin A, Cytarabin, Dacarbazin, Daclizumab, Dactinomycin, Dapson, Daunorubicin, Diclofenac, 1,11-Dimethoxycanthin-6-on, Docetaxel, Doxorubicin, Daunamycin, Epirubicin, Erythromycin, Estramustin, Etoposid, Everolimus, Filgrastim, Fluroblastin, Fluvastatin, Fludarabin, Fludarabin-5'-dihydrogenphosphat, Fluorouracil, Folimycin, Fosfestrol, Gemcitabin, Ghalakinosid, Ginkgol, Ginkgolsäure, Glykosid 1a, 4-Hydroxyoxycyclophosphamid, Idarubicin, Ifosfamid, Josamycin, Lapachol, Lomustin, Lovastatin, Melphalan, Midecamycin, Mitoxantron, Nimustin, Pitavastatin, Pravastatin, Procarbazin, Mitomycin, Methotrexat, Mercaptopurin, Thioguanin, Oxaliplatin, Irinotecan, Topotecan, Hydroxycarbamid, Miltefosin, Pentostatin, Pegaspargase, Exemestan, Letrozol, Formestan, Mycophenolatmofetil, β-Lapachon, Podophyllotoxin, Podophyllsäure-2-ethylhydrazid, Molgramostim (rhuGM-CSF), Peginterferon α-2b, Lenograstim (r-HuG-CSF), Macrogol, Selectin (Cytokinantagonist), Cytokinininhibitoren, COX-2-Inhibitor, Angiopeptin, monoklonale Antikörper, die die Muskelzellproliferation hemmen, bFGF-Antagonisten, Probucol, Prostaglandine, 1-Hydroxy-11-methoxycanthin-6-on, Scopoletin, NO-Donoren, Pentaerythrityltetranitrat und Sydnoimine, Tamoxifen, Staurosporin, β-Estradiol, α-Estradiol, Estriol, Estron, Ethinylestradiol, Medroxyprogesteron, Estradiolcypionate, Estradiolbenzoate, Tranilast, Kamebakaurin und andere Terpenoide, die in der Krebstherapie eingesetzt werden, Verapamil, Tyrosinkinase-Inhibitoren (Tyrphostine), Paclitaxel, 6-α-Hydroxypaclitaxel, Taxotere, Mofebutazon, Lonazolac, Lidocain, Ketoprofen, Mefenaminsäure, Piroxicam, Meloxicam, Penicillamin, Hydroxychlorochin, Natriumaurothiomalat, Oxaceprol, β-Sitosterol, Myrtecain, Polidocanol, Nonivamid, Levomenthol, Ellipticin, D-24851 (Calbiochem), Colcemid, Cytochalasin A-E, Indanocin, Nocodazol, Bacitracin, Vitronectin-Rezeptor Antagonisten, Azelastin, Guanidylcyclase-Stimulator, Gewebsinhibitor der Metallproteinase-1 und 2, freie Nukleinsäuren, Nukleinsäuren in Virenüberträger inkorporiert, DNA-und RNA-Fragmente, Plasminogen-Aktivator Inhibitor-1, Plasminogen-Aktivator Inhibitor-2, Antisense Oligonukleotide, VEGF-Inhibitoren, IGF-1, Wirkstoffe aus der Gruppe der Antibiotika, Cefadroxil, Cefazolin, Cefaclor, Cefoxitin, Tobramycin, Gentamicin, Penicilline, Dicloxacillin, Oxacillin, Sulfonamide, Metronidazol, Enoxaparin, Heparin, Hirudin, PPACK, Protamin, Prourokinase, Streptokinase, Warfarin, Urokinase, Vasodilatoren, Dipyramidol, Trapidil, Nitroprusside, PDGF-Antagonisten, Triazolopyrimidin, Seramin, ACE-Inhibitoren, Captopril, Cilazapril, Lisinopril, Enalapril, Losartan, Thioprotease-Inhibitoren, Prostacyclin, Vapiprost, Interferon α, β und γ, Histamin-Antagonisten, Serotonin-Blocker, Apoptose-Inhibitoren, Apoptose-Regulatoren, Halofuginon, Nifedipin, Tocopherol, Tranilast, Molsidomin, Teepolyphenole, Epicatechingallat, Epigallocatechingallat, Leflunomid, Etanercept, Sulfasalazin, Tetracyclin, Triamcinolon, Mutamycin, Procainimid, Retinolsäure, Quinidin, Disopyrimid, Flecainid, Propafenon, Sotalol, natürliche und synthetisch hergestellte Steroide wie Bryophyllin A, Inotodiol, Maquirosid A, Ghalakinosid, Mansonin, Streblosid, Hydrocortison, Betamethason, Dexamethason, nichtsteroidale Substanzen (NSAIDS), Fenoprofen, Ibuprofen, Indomethacin, Naproxen, Phenylbutazon, antivirale Agentien, Acyclovir, Ganciclovir Zidovudin, Clotrimazol, Flucytosin, Griseofulvin, Ketoconazol, Miconazol, Nystatin, Terbinafin, antiprotozoale Agenzien, Chloroquin, Mefloquin, Quinin, natürliche Terpenoide, Hippocaesculin, Barringtogenol-C21-angelat, 14-Dehydroagrostistachin, Agroskerin, Agrostistachin, 17-Hydroxyagrostistachin, Ovatodiolid, 4,7 Oxycycloanisomelsäure, Baccharinoide B1, B2, B3 and B7, Tubeimosid, Bruceantinosid C, Yadanzioside N und P, Isodeoxyelephantopin, Tomenphantopin A und B, Coronarin A,B C und D, Ursolsäure, Hyptatatsäure A, Iso-Iridogermanal, Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, Cymarin, Hydroxyanopterin, Protoanemonin, Cheliburinchlorid, Sinococulin A and B, Dihydronitidin, Nitidinchlorid, 12-β-Hydroxypregnadien-3,20-dion, Helenalin, Indicin, Indicin-N-oxid, Lasiocarpin, Inotodiol, Podophyllotoxin, Justicidin A und B, Larreatin, Malloterin, Mallotochromanol, Isobutyrylmallotochromanol, Marchantin A, Maytansin, Lycoridicin, Margetin, Pancratistatin, Liriodenin, Oxoushinsunin, Periplocosid A, Deoxypsorospermin, Psychorubin, Ricin A, Sanguinarin, Manwuweizsäure, Methylsorbifolin, Chromone der Spathelia, Stizophyllin, Dihydrousambaraensin, Hydroxyusambarin, Strychnopentamin, Strychnophyllin, Usambarin, Usambarensin, Liriodenin, Daphnoretin, Lariciresinol, Methoxylariciresinol, Syringaresinol, Sirolimus (Rapamycin), Biolimus A9, Pimecrolimus, Everolimus, Zotarolimus, Tacrolimus, Fasudil, Epothilone, Somatostatin, Roxithromycin, Troleandomycin, Simvastatin, Rosuvastatin, Vinblastin, Vincristin, Vindesin, Teniposid, Vinorelbin, Trofosfamid, Treosulfan, Temozolomid, Thiotepa, Tretinoin, Spiramycin, Umbelliferon, Desacetylvismion A, Vismion A und B, Zeorin.

**7.** Katheterballon gemäß Anspruch 6, wobei der Wirkstoff ausgewählt ist aus der Gruppe bestehend aus:

Paclitaxel, Taxane, Docetaxel, Rapamycin, Biolimus A9, Pimecrolimus, Everolimus, Zotarolimus, Tacrolimus, Fasudil und Epothilone.

**8.** Katheterballon gemäß Anspruch 7, wobei der Wirkstoff Paclitaxel ist.

**9.** Verfahren zur Beschichtung eines Katheterballons gemäß Anspruch 1 umfassend folgende Schritte:

A) Bereitstellen eines unbeschichteten Katheterballons; und
B) Bereitstellen einer Lösung eines Wirkstoffs und Bereitstellen einer Schellack-Lösung; und
C) Beschichten der Oberfläche des Katheterballons mit der wässrigen Schellack-Lösung; und
D) Auftragen der Lösung des Wirkstoffs, so dass zwei verschiedene Konzentrationsgradienten des Wirkstoffs in vertikaler und horizontaler Richtung im Schellack mit einem Gradientenmischer induziert werden; und anschließend
E) Trocknen des beschichteten Katheterballons.

**10.** Verfahren gemäß Anspruch 9, wobei die Lösung des Wirkstoffs mit einem Lösungsmittel hergestellt wird, wobei die Löslichkeit des Wirkstoffs, vorzugsweise Paclitaxel größer als die des Schellacks ist.

**11.** Verfahren gemäß Anspruch 9 oder 10, wobei die Lösung des Wirkstoffs in Ethylacetat hergestellt wird.

**12.** Verfahren gemäß einem der Ansprüche 9 bis 11, wobei der Wirkstoff Paclitaxel ist.

**13.** Verfahren gemäß einem der Ansprüche 9 bis 12, wobei die Wirkstoff-enthaltende Lösung mittels Sprühbeschichtung, Streichbeschichtung, Gasphasenabscheidung oder Pipettieren aufgetragen wird.

**14.** Dilatationskatheter umfassend den beschichteten Katheterballon gemäß einem der Ansprüche 1 - 7.

## Revendications

**1.** Un ballonnet à cathéter comprenant un revêtement avec un agent actif et shellac,
dans lequel le revêtement comprend un gradient vertical de concentration de l'agent actif et un gradient horizontal de l'agent actif, où pour le gradient vertical de concentration, la concentration de l'agent actif augmente de la surface du ballonnet jusqu'en haut ou jusqu'à la surface du revêtement.

**2.** Le ballonnet à cathéter selon la revendication 1, où le gradient de concentration de l'agent actif est dans la couche de shellac en tant que substance de matrice.

**3.** Le ballonnet à cathéter selon la revendication 1 ou 2, où le revêtement comprend en outre une couche de base de shellac comme première couche en dessous du gradient de concentration de l'agent actif.

**4.** Le ballonnet à cathéter selon une quelconque des revendications 1 - 3, où le revêtement comprend en outre une couche supérieure de shellac.

**5.** Le ballonnet à cathéter selon une quelconque des revendications 1 - 4, où l'agent actif est un agent antiprolifératif, un agent immunosuppresseur, un agent anti-angiogénique, un agent anti-inflammatoire, un agent anti-resténotique, et/ou un agent anti-thrombotique.

**6.** Le ballonnet à cathéter selon une quelconque des revendications 1 - 5, où l'agent actif est choisi à partir du groupe constitué par:

abciximab, acemétacine, acétylvismione B, aclarubicine, ademétionine, adriamycine, aescin, afromosone, akagerine, aldesleukine, amidorone, aminoglutéthimide, amsacrine, anakinra, anastrozole, anémonin, anopterine, antimycotiques, antithrombotiques, apocymarine, argatroban, aristolactame-AII, acide aristolochique, ascomycine, asparaginase, aspirine, atorvastatine, auranofine, azathioprine, azithromycine, baccatin, bafilomycine, basiliximab, bendamustine, benzocaine, berbérine, bétuline, acide bétulinique, bilobol, bisparthenolidine, bléomycine, combrestatin, acides boswelliques, brucéanol A, B et C, bryophylline A, busulfan, antithrombine, bivalirudine, cadhérines, camptothécine, capécitabine, acide o-carbamoyl-phenoxyacétique, carboplatine, carmustine, célécoxib, cépharanthine, cérivastatine, inhibiteurs de la CETP, chlorambucil, chloroquine phosphate, cicutoxine, ciprofloxacine, cisplatine, cladribine, clarithromycine, colchicine, concanamycine, coumadin, peptide natriurétique de type C (CNP), cudraisoflavone A, curcumine, cyclophosphamide, ciclosporine A, cytarabine, dacarbazine, daclizumab, dactinomycin, dapsone, daunorubicine, diclofenac, 1,11-diméthoxycanthin-6-one, docétaxel, doxorubicine, daunamycine, épirubicine, érythromycine, éstramustine, étoposide, everolimus, filgrastim, fluroblastin, fluvastatin, fludarabine, fludarabine-5'-dihydrogéne phosphate, fluorouracile, folimycine, fosfestrol, gemcitabine, ghalakinoside, ginkgol, acide ginkgolique, glycoside 1a, 4-hydroxyoxycyclo phosphamide, idarubicine, ifosfamide, josamycine, lapachol, lomustine, lovastatin, melphalan, midécamycine, mitoxantrone, nimustine, pitavastatine, pravastatine, procarbazine, mitomycine, méthotrexate, mércaptopurine, thioguanine, oxaliplatin, irinotécan, topotécan, hydroxycarbamide, miltéfosine, pentostatine, pegaspargase, exémestane, létrozole, formestane, mycophénolate mofétil, β-lapachone, podophyllotoxine, acide podophyllique-2-éthylhydrazide, molgramostim (rhuGM-CSF), pegintérferon α-2b, lenograstim (r-HuG-CSF), macrogol, sélectine (cytokine antagoniste), inhibiteurs des cytokines, inhibiteur de la COX-2, angiopeptine, anticorps monoclonaux inhibiteurs de la prolifération des cellules musculaires, antagonistes du bFGF, probucol, prostaglandines, 1-hydroxy-11-méthoxycanthin-6-one, scopolétol, donneurs de NO, pentaérythrityl tetranitrate et sydnoimines, dérivés S-nitroso, tamoxifène, staurosporine, β-estradiol, α-estradiol, estriol, estrone, éthinyl estradiol, médroxyprogestérone, cypionates d'estradiol, benzoates d'estradiol, tranilast, kamebakaurine et autres terpénoides utilisés dans la thérapie du cancer, verapamil, inhibiteurs de la tyrosine kinase (tyrphostins), paclitaxel, 6-a-hydroxy-paclitaxel, taxotères, mofebutazone, lonazolac, lidocaine, kétoprofène, acide méfénamique, piroxicam, méloxicam, pénicillamine, hydroxychloroquine, aurothiomalate de sodium, oxacéprol, β-sitostérol, myrtécaïne, polidocanol, nonivamide, lévomenthol, ellipticine, D-24851 (Calbiochem), colcémid, cytochalasines A-E, indanocine, nocodazole, bacitracine, antagonistes du récepteur de la vitronectine, azélastine, stimulateur de la guanidyl cyclase, inhibiteur tissulaire des métalloprotéinases 1 et 2, acides nucléiques libres, acides nucléiques incorporés dans des virus transmetteurs, fragments de l'ADN et l'ARN, inhibiteur-1 de l'activateur du plasminogène, inhibiteur-2 de l'activateur du plasminogène, les oligonucléotides antisens, les inhibiteurs du VEGF, l'IGF-1, agents actifs du groupe des antibiotiques, céfadroxil, céfazolin, céfaclor, céfoxitin, tobramycine, gentamicine, pénicillines, dicloxacilline, oxacilline, sulfonamides, métronidazole, énoxaparin, héparine, hirudine, PPACK, protamine, prourokinase, streptokinase, warfarine, urokinase, vasodilatateurs, dipyramidole, trapidil, nitroprussides, antagonistes du PDGF, triazolopyrimidine, seramin, inhibiteurs de l'ACE, captopril, cilazapril, lisinopril, énalapril, losartan, inhibiteurs de la thioprotéase, prostacycline, vapiprost, interféron α, β et γ, antagonistes d'histamine, bloqueurs de la sérotonine, inhibiteurs de l'apoptose, régulateurs de l'apoptose, halofuginone, nifédipine, tocophérol, tranilast, molsidomine, polyphénols du thé, gallate d'épicatéchine, gallate d'épigallocatéchine, léflunomide, étanercept, sulfasalazine, tétracycline, triamcinolone, mutamycine, procainimide, acide rétinoïque, quinidine, disopyrimide, flécaïnide, propafénone, sotalol, stéroïdes naturels et synthétiques comme la bryophylline A, inotodiol, maquiroside A, ghalakinoside, mansonine, strebloside, hydrocortisone, bétaméthasone, déxaméthasone, substances non-stéroïdiennes (NSAIDS), fénoprofène, ibuprofène, indomèthacine, naproxen, phénylbutazone, agents antiviraux, acyclovir, ganciclovir, zidovudine, clotrimazole, flucytosine, griséofulvine, kétoconazole, miconazole, nystatin, terbinafine, agents antiprotozoaires, chloroquine, méfloquine, quinine, terpénoïdes naturels, hippocaesculine, barringtogénol-C21-angelate, 14-déhydroagrostistachine, agroskerine, agrostistachine, 17-hydroxyagrostistachine, ovatodiolids, acide 4,7-oxycycloanisomelique, baccharinoids B1, B2, B3 et B7, tubeimoside, bruceantinoside C, yadanziosides N et P, isodéoxyelephantopine, tomenphantopine A et B, coronarine A, B, C et D, acide ursolique, acide hyptatique A, iso-iridogermanal, maytenfoliol, effusantine A, excisanine A et B, longikaurine B, sculponeatin C, kamebaunin, leukamenin A et B, 13,18-déhydro-6-alpha-senecioyloxychaparrin, taxamairin A et B, regenilol, triptolide, cymarin, hydroxyanoptérine, protoanémonin, chlorure de cheliburin, sinococuline A et B, dihydronitidine, chlorure de nitidine, 12-β-hydroxypregnadièn-3,20-dione, hélénaline, indicine, indicine-N-oxide, lasiocarpine, inotodiol, podophyllotoxin, justicidine A et B, larréatin, malloterin, mallotochromanol, isobutyrylmallotochromanol, marchantine A, maytansine, lycoridicine, margétine, pancratistatin, liriodènine, oxoushinsunine, periplocoside A, déoxypsorospermine, psychorubine, ricin A, sanguinarine, acide du blé manwu, méthylsorbifoline, chromones de spathelia, stizophylline, dihydro usambaraensine, hydroxyusambarine, strychnopentamine, strychnophylline, usambarine, usambarensine, liriodenine, daphnoretine, laricirésinol, méthoxylaricirésinol, syringarésinol, sirolimus (rapamycine),

biolimus A9, pimécrolimus, éverolimus, zotarolimus, tacrolimus, fasudil, épothilones, somatostatine, roxithromycine, troleandomycine, simvastatine, rosuvastatine, vinblastine, vincristine, vindesine, teniposide, vinorelbine, trofosfamide, tréosulfan, témozolomide, thiotépa, trétinoine, spiramycine, umbellifèrone, desacétylvismione A, vismione A et B, zeorin.

**7.** Le ballonnet à cathéter selon la revendication 6, où l'agent actif est choisi à partir du groupe constitué par:

paclitaxel, taxanes, docétaxel, rapamycine, biolimus A9, pimécrolimus, éverolimus, zotarolimus, tacrolimus, fasudil et épothilones

**8.** Le ballonnet à cathéter selon la revendication 7, où l'agent actif est paclitaxel.

**9.** Une méthode pour le revêtement d'un ballonnet à cathéter selon la revendication 1 comprenant les étapes suivantes:

A) fournir un ballonnet à cathéter non revêtu ; et
B) fournir une solution d'un agent actif et une solution de shellac; et
C) revêtir la surface du ballonnet à cathéter avec la solution de shellac; et
D) appliquer la solution de l'agent actif afin que deux diffèrent gradients de concentration de l'agent actif en direction verticale et en direction horizontale sont induits en utilisant un mélangeur de gradient ; et ultérieurement
E) sécher le ballonnet à cathéter revêtu.

**10.** La méthode selon la revendication 9, dans laquelle la solution d'agent actif est préparée en utilisant un solvant dans lequel la solubilité de l'agent actif, de préférence paclitaxel, est supérieure à celle de shellac.

**11.** La méthode selon la revendication 9 ou 10, dans laquelle la solution d'agent actif est préparée en acétate d'éthyle.

**12.** La méthode selon une quelconque des revendications 9 à 11, dans laquelle l'agent actif est le paclitaxel.

**13.** La méthode selon une quelconque des revendications 9 à 12, dans laquelle la solution contenant l'agent actif est appliquée par revêtement par pulvérisation, revêtement à la brosse, dépôt en phase vapeur ou pipetage.

**14.** Un cathéter de dilatation comprenant le ballonnet à cathéter selon une quelconque des revendications 1 - 7.

Figure 1

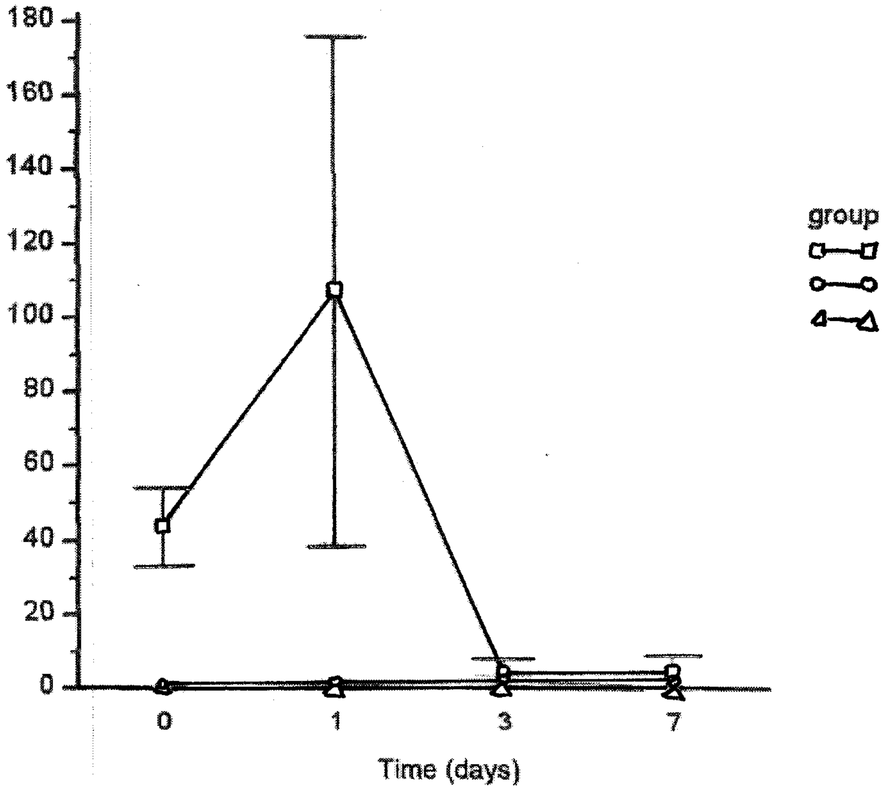


Figure 2

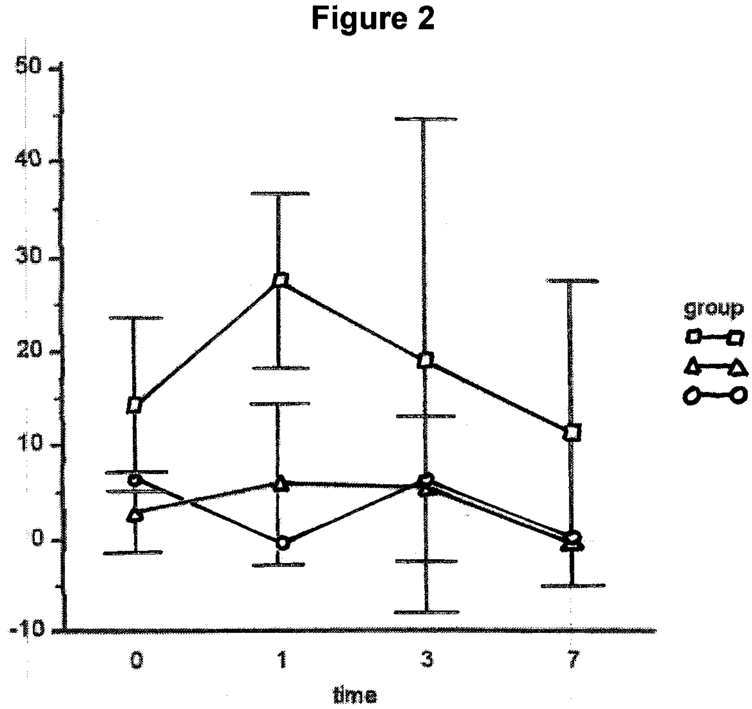

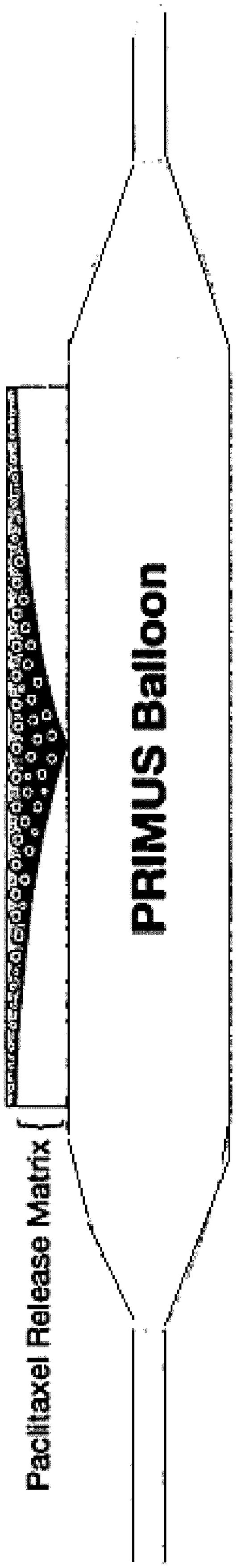


Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004028582 A1
- WO 2004006976 A1
- EP 2421572 A
- WO 9423787 A1
- WO 03059430 A1
- EP 0519063 B1
- WO 02043796 A2


### Non-patent literature cited in the description


- *Circulation,* 2004, vol. 110, 810-814
- **GRAY WA ; GRANADA JF.** Drug-coated balloons for the prevention of vascular restenosis. *Circulation,* vol. 121, 2672-80
- **POSA A et al.** Attainment of local drug delivery with paclitaxel-eluting balloon in porcine coronary arteries. *Coron Artery Dis,* 2008, vol. 19, 243-7
- **SCHELLER B ; SPECK U ; SCHMITT A ; BOHM M ; NICKENIG G.** Addition of paclitaxel to contrast media prevents restenosis after coronary stent implantation. *J Am Coll Cardiol,* 2003, vol. 42 (141), 5-20